(19) Europäisches Patentamt
European Patent Office
Office européen des brevets

(11) EP 1 518 860 A1

(12) DEMANDE DE BREVET EUROPEEN

(43) Date de publication:
**30.03.2005 Bulletin 2005/13**

(51) Int Cl.[7]: **C07D 521/00**

(21) Numéro de dépôt: **04292289.8**

(22) Date de dépôt: **23.09.2004**

(84) Etats contractants désignés:
**AT BE BG CH CY CZ DE DK EE ES FI FR GB GR HU IE IT LI LU MC NL PL PT RO SE SI SK TR**
Etats d'extension désignés:
**AL HR LT LV MK**

(30) Priorité: **26.09.2003 FR 0311307**

(71) Demandeur: **L'OREAL**
**75008 Paris (FR)**

(72) Inventeurs:
- **Vidal, Laurent**
**75013 Paris (FR)**
- **Sabelle, Stéphane**
**75005 Paris (FR)**
- **Ly-Carry, Thi-My**
**94100 St. Maur (FR)**

(74) Mandataire: **Lhoste, Catherine**
**L'OREAL - D.I.P.I.**
**25-29 Quai Aulagnier**
**92600 Asnieres (FR)**

# (54) Dérivés de triazacyclononane substitué sur l'un au moins des atomes d'azote par un groupement 4-aminophényle substitué ou non substitué pour la teinture des fibres kératiniques

(57) L'invention a pour objet des dérivés de triazacyclononane substitué sur l'un au moins des atomes d'azote par un groupement 4'-aminophényle substitué ou non substitué, ainsi que leur utilisation à titre de base d'oxydation pour la teinture des fibres kératiniques, et en particulier des fibres kératiniques humaines telles que les cheveux. L'invention a aussi pour objet une composition pour la teinture des fibres kératiniques, et en particulier des fibres kératiniques humaines telles que les cheveux, comprenant au moins un dérivé de triazacyclononane substitué sur l'un au moins des atomes d'azote par un groupement 4'-aminophényle à titre de base d'oxydation, ainsi que le procédé de teinture mettant en oeuvre cette composition.

La présente invention permet en particulier d'obtenir une coloration des fibres kératiniques puissante, peu sélective et tenace.

EP 1 518 860 A1

## Description

L'invention a pour objet des dérivés de triazacyclononane substitué sur l'un au moins des atomes d'azote par un groupement 4'-aminophényle substitué ou non substitué, ainsi que leur utilisation à titre de base d'oxydation pour la teinture des fibres kératiniques, et en particulier des fibres kératiniques humaines telles que les cheveux.

Il est connu de teindre les fibres kératiniques, et en particulier les fibres kératiniques humaines telles que les cheveux avec des compositions tinctoriales comprenant des précurseurs de colorant d'oxydation, en particulier des ortho ou para-phénylènediamines, des ortho ou para-aminophénols, des composés hétérocycliques tels que des dérivés de diaminopyrazole, des dérivés de pyrazolo[1,5-a]pyrimidine, des dérivés de pyrimidine, des dérivés de pyridine, des dérivés de 5,6-dihydroxyindole, des dérivés de 5,6-dihydroxyindoline appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés ou colorants.

On sait également que l'on peut faire varier les nuances obtenues avec ces bases d'oxydation en les associant à des coupleurs ou modificateurs de coloration, ces derniers étant choisis notamment parmi les méta-phénylènediamines, les méta-aminophénols, les méta-hydroxyphénols et certains composés hétérocycliques tels que par exemple des dérivés de pyrazolo[1,5-b]-1,2,4-triazole, des dérivés de pyrazolo[3,2-c]-1,2,4-triazole, des dérivés de pyrazolo [1,5-a]pyrimidine, des dérivés de pyridine, des dérivés de pyrazol-5-one, des dérivés d'indoline et des dérivés d'indole.

La variété des molécules mises en jeu au niveau des bases d'oxydation et des coupleurs permet l'obtention d'une riche palette de couleurs.

La coloration dite "permanente" obtenue grâce à ces colorants d'oxydation doit par ailleurs satisfaire un certain nombre d'exigences. Ainsi, elle doit être sans inconvénient sur le plan toxicologique, elle doit permettre d'obtenir des nuances dans l'intensité souhaitée, présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui peut être en effet différemment sensibilisée (i.e. abîmée) entre sa pointe et sa racine. Ils doivent également présenter une bonne stabilité chimique dans les formulations. Ils doivent présenter un bon profil toxicologique.

Dans le domaine de la coloration capillaire, la para-phénylènediamine et la para-toluènediamine sont des bases d'oxydation largement utilisées. Elles permettent d'obtenir avec des coupleurs d'oxydation des nuances variées.

Cependant, il existe un besoin de découvrir de nouvelles bases d'oxydation présentant un meilleur profil toxicologique que la para-phénylènediamine et la para-toluènediamine, tout en permettant de conférer aux cheveux d'excellentes propriétés d'intensité de couleur, de variété de nuances, d'uniformité de la couleur et de ténacité aux agents extérieurs.

Il est déjà connu d'utiliser des dérivés 1,4-diazacycloheptane pour la coloration d'oxydation des fibres kératiniques. Par exemple, le brevet US 6 165 230 décrit une composition tinctoriale comprenant des dérivés 1,4-diazacyctoheptane substitués sur les deux atomes d'azote du cycle par un groupement 4'-aminophényle.

Il est également connu d'utiliser des dérivés 1,4,7-triazacyclononane pour la coloration des fibres kératiniques. Par exemple, le brevet JP 2002-255763 décrit une composition tinctoriale comprenant des dérivés 1,4,7-triazacyclononane substitués sur un ou plusieurs atomes d'azote du cycle par un groupement 2'-aminobenzyle.

Il est clairement établi que ces composés ne permettent pas de conférer une coloration de qualité équivalente à celle obtenue avec la para-phénylènediamine ou avec la para-toluènediamine du fait d'un manque d'intensité et d'uniformité de la couleur.

Il existe donc un réel besoin de découvrir de nouvelles bases d'oxydation présentant à la fois un bon profil toxicologique et des propriétés telles que les compositions tinctoriales les comprenant permettent de conférer aux cheveux d'excellentes propriétés d'intensité de couleur, de variété de nuances, d'uniformité de la couleur et de ténacité vis-à-vis des différentes agressions extérieures que peuvent subir les cheveux.

Le but de la présente invention est de développer de nouvelles bases d'oxydation pour la teinture des fibres kératiniques ne présentant pas les inconvénients des bases d'oxydation de la technique antérieure en fournissant de nouveaux composés pour la teinture des fibres kératiniques qui ne dégradent pas les fibres kératiniques tout en étant capables d'engendrer des colorations intenses dans des nuances variées, peu sélectives, particulièrement résistantes et présentant un bon profil toxicologique.

Ce but est atteint avec la présente invention qui a pour objet les dérivés de triazacyclononane substitué sur l'un au moins des atomes d'azote par un groupement 4'-aminophényle substitué ou non substitué.

La présente invention permet en particulier d'obtenir une coloration des fibres kératiniques puissante, peu sélective et tenace.

Un autre objet de l'invention est l'utilisation de ces dérivés de triazacyclononane à titre de base d'oxydation

pour la teinture des fibres kératiniques, et en particulier des fibres kératiniques humaines telles que les cheveux.

L'invention a aussi pour objet une composition pour la teinture des fibres kératiniques, et en particulier des fibres kératiniques humaines telles que les cheveux, comprenant au moins un dérivé de triazacyclononane tel que défini précédemment à titre de base d'oxydation, ainsi que le procédé de teinture des fibres kératiniques mettant en oeuvre cette composition.

L'invention a enfin pour objet les dérivés de triazacyclononane substitué sur l'un au moins des atomes d'azote par un groupement 4'-nitrophényle substitué ou non substitué permettant de synthétiser les dérivés de triazacyclononane tels que définis précédemment.

Selon un mode de réalisation particulier de l'invention, les dérivés de triazacyclononane substitué sur l'un au moins des atomes d'azote par un groupement 4'-aminophényle substitué ou non substitué sont choisis parmi les composés de formule (I) suivante et leurs sels d'addition :

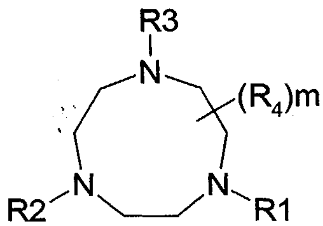

(I)

dans laquelle :

- $R_1$, $R_2$ et $R_3$ représentent :
  - un atome d'hydrogène ;
  - un radical alkyle pouvant être saturé ou insaturé, non substitué ou substitué par un ou plusieurs radicaux carboxyle, alkylcarbonyle, alcoxycarbonyle, carbamoyle, mono- ou di-alkylcarbamoyle, alkylsulfonyle, trialkylammonium, N-alkylimidazolium, hétérocycliques à 4, 5, 6 ou 7 atomes, saturés ou insaturés, dont le ou les hétéroatomes sont choisis parmi l'azote, l'oxygène et le soufre ;
  - un radical $-CH_2-R'$ où R' est un radical alkyle pouvant être saturé ou insaturé, substitué par un ou plusieurs radicaux hydroxy, alcoxy, amino, mono- ou di-alkylamino, trialkylammonium, alkylcarbonylamino, alkylcarbonyloxy ;
  - un radical alkylcarbonyle pouvant être saturé ou insaturé, non substitué ou substitué par un ou plusieurs radicaux hydroxyle, alcoxy, amino, mono- ou di-alkylamino, alkylsulfonyle, carboxyle, alkylcarbonyle, alcoxycarbonyle, carbamoyle, mono- ou di-alkylcarbamoyle, trialkylammonium, N-alkylimidazolium, hétérocycliques à 4, 5, 6 ou 7 atomes, saturés ou insaturés, dont le ou les hétéroatomes sont choisis parmi l'azote, l'oxygène et le soufre ;
  - un radical alcoxycarbonyle ;
  - un radical mono- ou di-alkylcarbamoyle ;
  - un radical carbamoyle ;
  - un radical carboxyle ;
  - un radical alkylsulfonyle ;
  - un radical arylsulfonyle ;
  - un radical correspondant à la formule (II) :

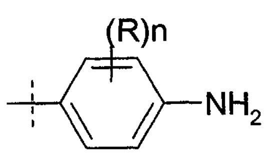

(II)

dans laquelle :

- R représente :

- un radical alkyle pouvant être saturé ou insaturé, non substitué ou substitué par un ou plusieurs radicaux hydroxyle, amino, guanidinyle, trialkylammonium, N-alkylimidazolium, carboxyle, carbamoyle ;
- un radical alcoxy pouvant être saturé ou insaturé, non substitué ou substitué par un ou plusieurs radicaux hydroxyle, amino, trialkylammonium, N-alkylimidazolium ;
- un radical alcoxyalkyle ;
- un radical hydroxyalcoxyalkyle ;
- un radical aminoalkyle, l'amine pouvant être non substituée ou mono ou disubstituée par un radical alkyle, acétyle, hydroxyalkyle ;

• n est compris entre 0 et 4, étant entendu que lorsque n est supérieur à 1 alors les radicaux R peuvent être identiques ou différents ;

étant entendu qu'au moins un des radicaux $R_1$, $R_2$ ou $R_3$ est choisi parmi les radicaux de formule (II) ;

• $R_4$ représente :

- un radical alkyle pouvant être saturé ou insaturé ;
- un radical hydroxyalkyle ;
- un radical alcoxyalkyle ;
- un radical alkylcarbonyle ;
- un radical hydroxyalcoxyalkyle ;
- un radical aminoalkyle, l'amine pouvant être non substituée ou mono ou disubstituée par un radical alkyle, acétyle, hydroxyalkyle ;
- un radical hydroxy et aminoalkyle ;
- un radical carboxyle ;
- un radical carboxyalkyle ;
- un radical carbamoyle ;
- un radical carbamoylalkyle ;
- un radical alcoxycarbonyle ;
- un radical mono- ou di-alkylaminocarbonyle ;

• m est compris entre 0 et 6, étant entendu que lorsque m est supérieur à 1 alors les radicaux $R_4$ peuvent être identiques ou différents.

Dans les définitions ci-dessus, les radicaux alkyle sont linéaires ou ramifiés et comprennent, sauf autre indication, de 1 à 10 atomes de carbone, de préférence de 1 à 6 atomes de carbone. Un radical alcoxy est un radical alkyl-O-, le radical alkyle étant tel que défini précédemment.

On entend par radical alkyle insaturé, un radical alkyle de 2 à 10 atomes de carbone et comprenant une ou plusieurs doubles et / ou triples liaisons.

Un radical alkyle substitué est un radical alkyle mono ou polysubstitué. Par exemple, un radical hydroxyalkyle ou un radical aminoalkyle est un radical alkyle qui peut être substitué par un ou plusieurs groupements hydroxy ou amino.

Lorsque les dérivés de formule (I) sont cationiques, par exemple lorsque l'un des radicaux $R_1$, $R_2$ ou $R_3$ représente un radical alkyle substitué par un radical trialkylammonium, ils sont associés à un ou plusieurs contre-ions choisis parmi les anions minéraux tels que les halogénures, les hydroxydes, les sulfates, les hydrogénosulfates ou les anions organiques tels que les acétates, les citrates, les tartrates, les alkylsulfates pour lesquels la partie alkyle, linéaire ou ramifiée, est en $C_1$-$C_6$, les alkylsulfonates pour lesquels la partie alkyle, linéaire ou ramifiée, est en $C_1$-$C_6$, les arylsulfonates pour lesquels la partie aryle, de préférence phényle, est éventuellement substituée par un ou plusieurs radicaux alkyle en $C_1$-$C_4$.

Dans les dérivés de formule (I), le radical R est par exemple choisi parmi un radical méthyle ; un radical éthyle ; un radical isopropyle ; un radical vinyle ; un radical allyle ; un radical méthoxyméthyle ; un radical hydroxyméthyle ; un radical 1-carboxyméthyle ; un radical 1-aminométhyle ; un radical 2-carboxyéthyle ; un radical 2-hydroxyéthyle ; un radical 3-hydroxypropyle ; un radical 1,2-dihydroxyéthyle ; un radical 1-hydroxy-2-aminoéthyle ; un radical 1-amino-2-hydroxyéthyle ; un radical 1,2-diaminoéthyle ; un radical méthoxy ; un radical éthoxy ; un radical allyloxy ; un radical 2-hydroxyéthyloxy.

Selon un mode de réalisation particulier de l'invention, deux des radicaux $R_1$, $R_2$ ou $R_3$ sont des radicaux correspondant à la formule (II).

Selon un mode de réalisation particulier de l'invention, n est égal à 0 ou R est choisi parmi un radical alkyle ; un radical hydroxyalkyle ; un radical aminoalkyle ; un radical alcoxy ; un radical hydroxyalcoxy. A titre d'exemple, R

peut être un radical méthyle ; un radical hydroxyméthyle ; un radical 1,2-dihydroxyéthyle.

Selon un mode de réalisation préféré de l'invention, n est égal à 0 ou 1.

Selon un mode de réalisation particulier de l'invention, le ou les radicaux $R_1$, $R_2$ ou $R_3$ différents des radicaux correspondant à la formule (II) sont choisis parmi un atome d'hydrogène ; un radical alkyle ; un radical alkylcarbonyle ; un radical alkylsulfonyle ; un radical $-CH_2-R'$ où R' est un radical alkyle substitué par un ou plusieurs radicaux hydroxy, alcoxy, amino, mono- ou di-alkylamino, trialkylammonium ; un radical alkyle substitué par un ou plusieurs radicaux trialkylammonium, N-alkylimidazolium ; un radical alkylcarbonyle substitué par un ou plusieurs radicaux N-alkylimidazolium ; un radical mono- ou di-alkylcarbamoyle. A titre d'exemple, le ou les radicaux $R_1$, $R_2$ ou $R_3$ différents des radicaux correspondant à la formule (II) peuvent être un atome d'hydrogène ; un radical 2-hydroxyéthyle ; un radical 2,3-dihydroxy-propyle; un radical 3-hydroxypropyle ; un radical 2-aminoéthyle ; un radical 2-méthoxyéthyle ; un radical méthyle ; un radical éthyle ; un radical acétyle ; un radical méthylsulfonyle ; un radical N,N-diméthyl-carbamoyle; un radical 2-(N,N-diméthylamino)éthyle ; un radical (1-méthyl-imidazol-1-ium-3-yl)méthylcarbonyle ; un radical 3-(triméthylammonium)-2-hydroxy-propyle, un radical (1-méthyl-imidazol-1-ium-3-yl)propyle ; un radical 2-(triméthylammonium)-éthyle.

Selon un mode de réalisation particulier de l'invention, m est égal à 0 ou $R_4$ est choisi parmi un radical alkyle ; un radical hydroxyalkyle ; un radical carboxyle ; un radical carbamoyle.

Selon un mode de réalisation préféré de l'invention, m est égal à 0 ou 1.

Les composés de triazacyclononane tels que définis précédemment peuvent être éventuellement salifiés par des acides minéraux forts tels que par exemple HCl, HBr, $H_2SO_4$, ou des acides organiques tels que, par exemple, l'acide acétique, lactique, tartrique, citrique ou succinique.

A titre d'exemples de dérivés de triazacyclononane conforme à l'invention, on peut citer les composés présentés dans le tableau ci-dessous.

| Formule | Nomenclature | formule | Nomenclature |
|---|---|---|---|
| 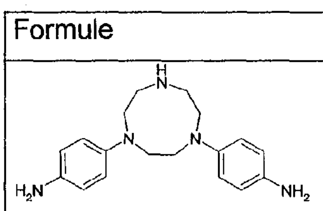 | 1,4-bis-(4-amino-phényl)-[1,4,7] triazonane | 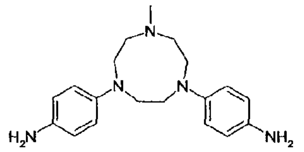 | 1-méthyl-4,7-bis-(4-amino-phényl)-[1,4,7] triazonane |
| 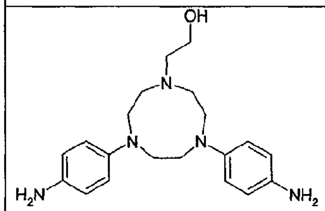 | 2-[4,7-bis-(4-amino-phényl)-[1,4,7]triazonan-1-yl]-éthanol | 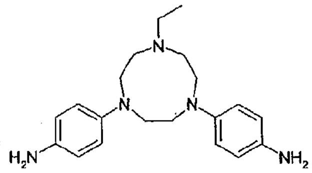 | 1-éthyl-4,7-bis-(4-amino-phényl)-[1,4,7] triazonane |
| 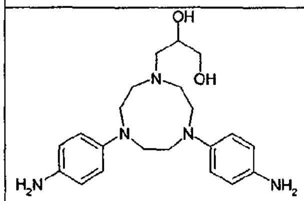 | 3-[4,7-bis-(4-amino-phényl)-[1,4,7]triazonan-1-yl]-propane-1,2-diol | 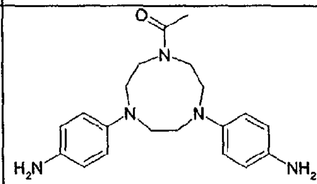 | 1-acétyl-4,7-bis-(4-amino-phényl)-[1,4,7] triazonane |
| 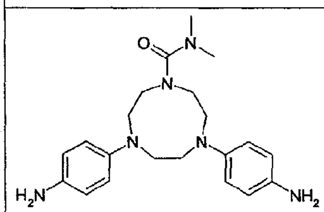 | 1-(N,N-diméthyl-carbamoyl)-4,7-bis-(4-amino-phényl)-[1,4,7] triazonane | 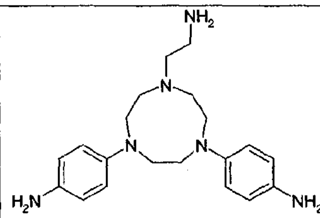 | 1-(β-amino-éthyl)-4,7-bis-(4-amino-phényl)-[1,4,7] triazonane |
| 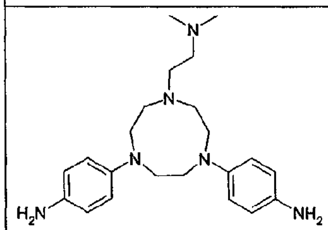 | 1-[β-(N,N-diméthyl-amino)-éthyl]-4,7-bis-(4-amino-phényl)-[1,4,7] triazonane | 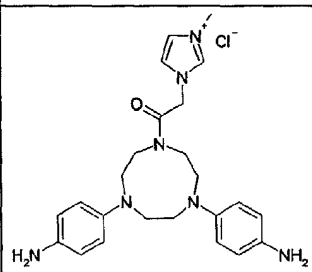 | Chlorure de 3-{2-[4,7-bis-(4-amino-phényl)-[1,4,7]triazonan-1-yl]-2-oxo-éthyl}-1-méthyl-3H-imidazol-1-ium |
| 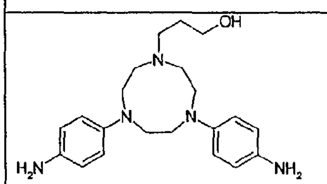 | 1-(γ-hydroxy-propyl)-4,7-bis-(4-amino-phényl)-[1,4,7] triazonane | 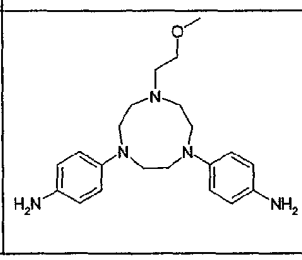 | 1-(β-méthoxy-éthyl)-4,7-bis-(4-amino-phényl)-[1,4,7] triazonane |

| | | | |
|---|---|---|---|
| 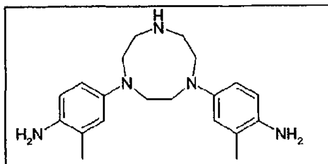 | 1,4-bis-(4-amino-3-méthyl-phényl)-[1,4,7] triazonane | 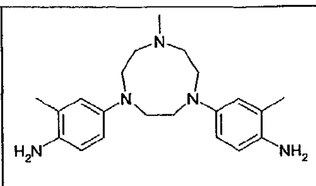 | 1-méthyl-4,7-bis-(4-amino-3-méthyl-phényl)-[1,4,7] triazonane |
| 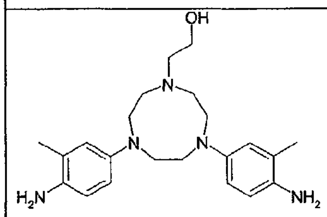 | 2-[4,7-bis-(4-amino-3-méthyl-phényl)-[1,4,7]triazonan-1-yl]-éthanol | 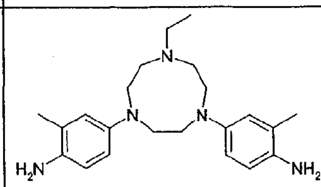 | 1-éthyl-4,7-bis-(4-amino-3-méthyl-phényl)-[1,4,7] triazonane |
| 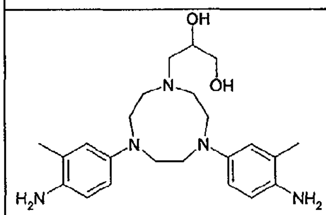 | 3-[4,7-bis-(4-amino-3-méthyl-phényl)-[1,4,7] triazonan-1-yl]-propane-1,2-diol | 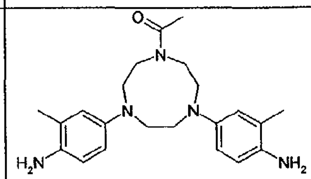 | 1-acétyl-4,7-bis-(4-amino-3-méthyl-phényl)-[1,4,7] triazonane |
| 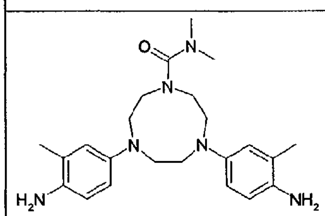 | 1-(N,N-diméthyl-carbamoyl)-4,7-bis-(4-amino-3-méthyl-phényl)-[1,4,7] triazonane | 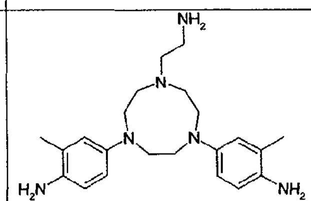 | 1-(β-amino-éthyl)-4,7-bis-(4-amino-3-méthyl-phényl)-[1,4,7] triazonane |
| 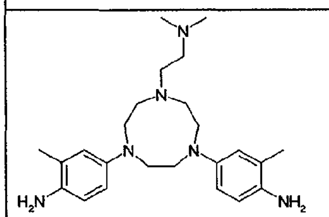 | 1-[β-(N,N-diméthyl-amino)-éthyl]-4,7-bis-(4-amino-3-méthyl-phényl)-[1,4,7] triazonane | 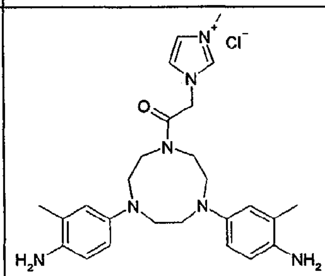 | Chlorure de 3-{2-[4,7-bis-(4-amino-3-méthyl-phényl)-[1,4,7]triazonan-1-yl]-2-oxo-éthyl}-1-méthyl-3H-imidazol-1-ium |
| 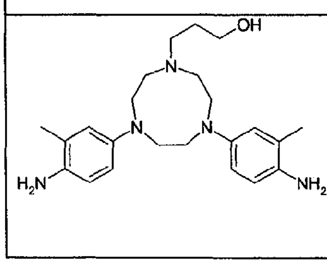 | 3-[4,7-bis-(4-amino-3-méthyl-phényl)-[1,4,7]triazonan-1-yl]-propan-1-ol | 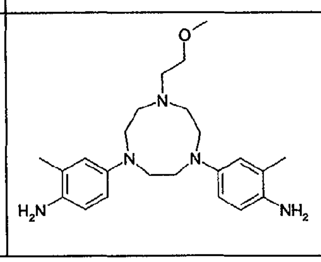 | 1-(β-méthoxy-éthyl)-4,7-bis-(4-amino-3-méthyl-phényl)-[1,4,7] triazonane |

| | | | |
|---|---|---|---|
| 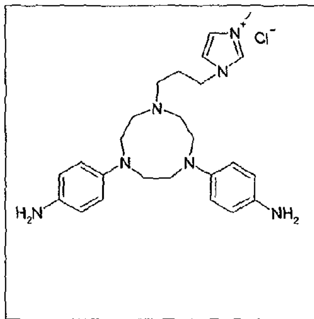 | Chlorure de 3-{3-[4,7-bis-(4-amino-phényl)-[1,4,7]triazonan-1-yl]-propyl}-1-méthyl-3H-imidazol-1-ium | 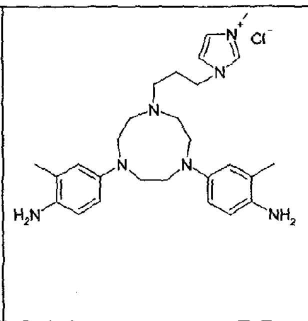 | Chlorure de 3-{3-[4,7-bis-(4-amino-3-méthyl-phényl)-[1,4,7]triazonan-1-yl]-propyl}-1-méthyl-3H-imidazol-1-ium |
| 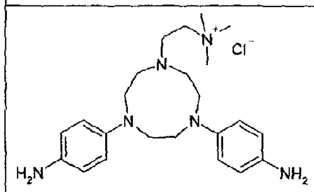 | Chlorure de {2-[4,7-bis-(4-amino-phényl)-[1,4,7]triazonan-1-yl]-éthyl}-triméthyl-ammonium | 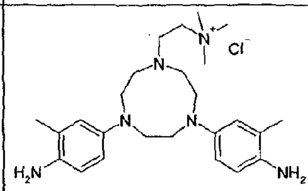 | Chlorure de {2-[4,7-bis-(4-amino-3-méthyl-phényl)-[1,4,7]triazonan-1-yl]-éthyl}-triméthyl-ammonium |
| 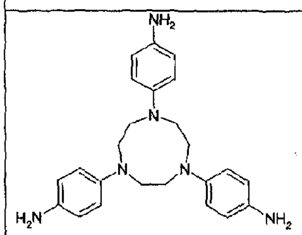 | 1,4,7-tris-(4-amino-phényl)-[1,4,7] triazonane | 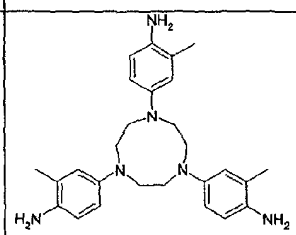 | 1,4,7-tris-(4-amino-3-méthyl-phényl)-[1,4,7] triazonane |
| 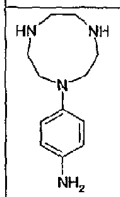 | 1-(4-amino-phényl)-[1,4,7] triazonane | 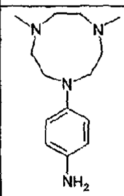 | 1,4-diméthyl-7-(4-amino-phényl)-[1,4,7] triazonane |
| 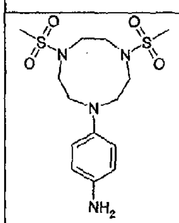 | 1,4-bis-méthanesulfonyl-7-(4-amino-phényl)-[1,4,7] triazonane | 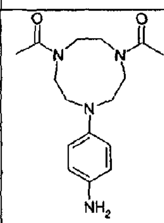 | 1,4-diacétyl-7-(4-amino-phényl)-[1,4,7] triazonane |
| 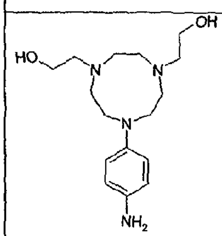 | 1,4-bis-(β-hydroxy-éthyl)-7-(4-amino-phényl)-[1,4,7] triazonane | 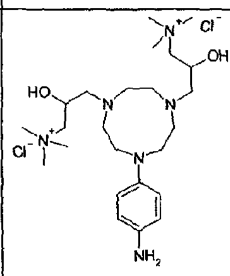 | 1-(4-amino-phényl)-4,7-bis-(γ-triméthyl-ammonium-β-hydroxy-propyl)-[1,4,7] triazonane |

| | | | |
|---|---|---|---|
| 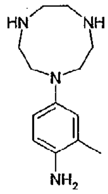 | 1-(4-amino-3-méthyl-phényl)-[1,4,7] triazonane | 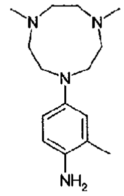 | 1,4-diméthyl-7-(4-amino-3-méthyl-phényl)-[1,4,7] triazonane |
| 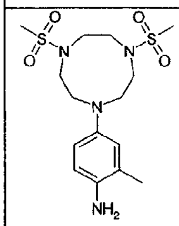 | 1,4-bis-méthanesulfonyl-7-(4-amino-3-méthyl-phényl)-[1,4,7] triazonane | 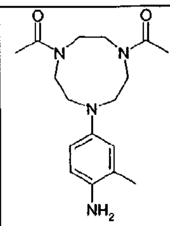 | 1,4-diacétyl-7-(4-amino-3-méthyl-phényl)-[1,4,7] triazonane |
| 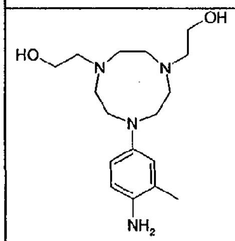 | 1,4-bis-(β-hydroxy-éthyl)-7-(4-amino-3-méthyl-phényl)-[1,4,7] triazonane | 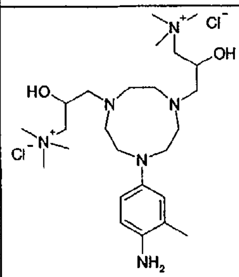 | Chlorure de 1,4-bis-(γ-triméthyl-ammonium-β-hydroxy-propyl)-7-(4-amino-3-méthyl-phényl)-[1,4,7] triazonane |

Parmi ces composés, les dérivés de formule (I) particulièrement préférés sont le 1-(4-amino-phényl)-[1,4,7] triazonane ; le 1,4-bis(4-amino-phényl)-[1,4,7]triazonane ; le 1,4,7-tris-(4-amino-phényl)-[1,4,7]triazonane ; le 2-[4,7-bis-(4-amino-phényl)-[1,4,7]triazonan-1-yl]-éthanol et le chlorure de 3-{3-[4,7-bis-(4-amino-phényl)-[1,4,7]triazonan-1-yl]-propyl}-1-méthyl-3H-imidazol-1-ium .

Le carbone substitué par $R_4$ peut être de configuration (R) et / ou (S).

La présente invention a également pour objet l'utilisation à titre de base d'oxydation pour la teinture d'oxydation des fibres kératiniques, et en particulier des fibres kératiniques humaines telles que les cheveux, d'un dérivé de triazacyclononane tel que défini précédemment.

La présente invention a également pour objet une composition pour la teinture des fibres kératiniques, et en particulier des fibres kératiniques humaines telles que les cheveux, comprenant, dans un milieu approprié pour la teinture, au moins un dérivé de triazacyclononane tel que défini précédemment à titre de base d'oxydation.

Le ou les dérivés de triazacyclononane conforme à l'invention sont en général présents chacun en quantité comprise entre 0,001 et 10 % en poids environ du poids total de la composition tinctoriale, de préférence entre 0,005 et 6 %.

La composition tinctoriale de l'invention peut contenir un ou plusieurs coupleurs conventionnellement utilisés pour la teinture de fibres kératiniques. Parmi ces coupleurs, on peut notamment citer les méta-phénylènediamines, les méta-aminophénols, les méta-diphénols, les coupleurs naphtaléniques, les coupleurs hétérocycliques et leurs sels d'addition.

A titre d'exemple, on peut citer le 2-méthyl 5-aminophénol ; le 5-N-(β-hydroxyéthyl)amino 2-méthyl phénol ; le 6-chloro-2-méthyl-5-aminophénol ; le 3-amino phénol ; le 1,3-dihydroxy benzène ; le 1,3-dihydroxy 2-méthyl benzène ; le 4-chloro 1,3-dihydroxy benzène ; le 2,4-diamino 1-(β-hydroxyéthyloxy) benzène ; le 2-amino 4-(β-hydroxyéthylamino) 1-méthoxybenzène ; le 1,3-diamino benzène ; le 1,3-bis-(2,4-diaminophénoxy) propane ; la 3-uréido aniline ; le 3-uréido 1-diméthylamino benzène ; le sésamol ; le 1-(β-hydroxyéthylamino)-3,4-méthylènedioxybenzène ; l'α-naphtol ; le 2-méthyl-1-naphtol ; le 6-hydroxy indole ; le 4-hydroxy indole ; le 4-hydroxy N-méthyl indole ; la 2-amino-3-hydroxy pyridine ; la 6-hydroxy benzomorpholine ; la 3,5-diamino-2,6-diméthoxypyridine ; le 1-N-(β-hydroxyéthyl)amino-3,4-méthylènedioxybenzène ; le 2,6-bis-(β-hydroxyéthylamino)toluène et leurs sels d'addition avec un acide.

Dans la composition de la présente invention, le ou les coupleurs sont chacun généralement présents en quantité comprise entre 0,001 et 10 % en poids environ du poids total de la composition tinctoriale, de préférence entre 0,005 et 6 %.

La composition de la présente invention peut en outre comprendre une ou plusieurs bases d'oxydation additionnelles classiquement utilisées en teinture d'oxydation autres que celles décrites précédemment. A titre d'exemple, ces bases d'oxydation additionnelles sont choisies parmi les para-phénylènediamines autres que les dérivés de triazacyclononane conforme à l'invention, les bis-phénylalkylènediamines, les para-aminophénols, les bis-para-aminophénols, les ortho-aminophénols, les ortho-phénylènediamines, les bases hétérocycliques et leurs sels d'addition.

Parmi les para-phénylènediamines, on peut citer à titre d'exemple, la para-phénylènediamine ; la para-toluènediamine ; la 2-chloro para-phénylènediamine ; la 2,3-diméthyl para-phénylènediamine ; la 2,6-diméthyl para-phénylènediamine ; la 2,6-diéthyl para-phénylènediamine ; la 2,5-diméthyl para-phénylènediamine ; la N,N-diméthyl para-phénylènediamine ; la N,N-diéthyl para-phénylènediamine ; la N,N-dipropyl para-phénylènediamine ; la 4-amino N,N-diéthyl 3-méthyl aniline ; la N,N-bis-(β-hydroxyéthyl) para-phénylènediamine ; la 4-N,N-bis-(β-hydroxyéthyl)amino 2-méthyl aniline ; la 4-N,N-bis-(β-hydroxyéthyl)amino 2-chloro aniline ; la 2-(β-hydroxyéthyl) para-phénylènediamine ; la 2-fluoro para-phénylènediamine ; la 2-isopropyl para-phénylènediamine ; la N-(β-hydroxypropyl) para-phénylènediamine ; la 2-hydroxyméthyl para-phénylènediamine ; la N,N-diméthyl 3-méthyl para-phénylènediamine ; la N,N-(éthyl, β-hydroxyéthyl) para-phénylènediamine ; la N-(β,γ-dihydroxypropyl) para-phénylènediamine ; la N-(4'-aminophényl) para-phénylènediamine ; la N-phényl para-phénylènediamine ; la 2-(β-hydroxyéthyloxy) para-phénylènediamine ; la 2-(β-acétylaminoéthyloxy) para-phénylènediamine ; la N-(β-méthoxyéthyl) para-phénylènediamine ; la 4-aminophényl pyrrolidine ; la 2-thiényl para-phénylènediamine ; la 2-(β-hydroxyéthylamino) 5-amino toluène, la 3-hydroxy 1-(4'-aminophényl) pyrrolidine et leurs sels d'addition avec un acide.

Parmi les para-phénylènediamines citées ci-dessus, la para-phénylènediamine ; la para-toluènediamine ; la 2-isopropyl para-phénylènediamine ; la 2-(β-hydroxyéthyl) para-phénylènediamine ; la 2-(β-hydroxyéthyloxy) para-phénylènediamine ; la 2,6-diméthyl para-phénylènediamine ; la 2,6-diéthyl para-phénylènediamine ; la 2,3-diméthyl para-phénylènediamine ; la N,N-bis-(β-hydroxyéthyl) para-phénylènediamine ; la 2-chloro para-phénylènediamine ; la 2-(β-acétylaminoéthyloxy) para-phénylènediamine et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol ; la N,N'-bis-(β-hydroxyéthyl) N,N'-bis-(4'-aminophényl) éthylènediamine ; la N,N'-bis-(4-aminophényl) tétraméthylènediamine ; la N,N'-bis-(β-hydroxyéthyl) N,N'-bis-(4-aminophényl) tétraméthylènediamine ; la N,N'-bis-(4-méthyl-aminophényl) tétraméthylènediamine ; la N,N'-bis-(éthyl) N,N'-(4'-amino, 3'-méthylphényl) éthylènediamine ; le 1,8-bis-(2,5-diaminophénoxy)-3,6-dioxaoctane et leurs sels d'addition avec un acide.

Parmi les para-aminophénols, on peut citer à titre d'exemple, le para-aminophénol ; le 4-amino 3-méthyl phénol ; le 4-amino 3-fluoro phénol ; le 4-amino 3-hydroxyméthyl phénol ; le 4-amino 2-méthyl phénol ; le 4-amino 2-hydroxyméthyl phénol ; le 4-amino 2-méthoxyméthyl phénol ; le 4-amino 2-aminométhyl phénol ; le 4-amino 2-(β-hydroxyéthylaminométhyl) phénol ; le 4-amino 2-fluoro phénol et leurs sels d'addition avec un acide.

Parmi les ortho-aminophénols, on peut citer à titre d'exemple le 2-amino phénol ; le 2-amino 5-méthyl phénol ; le 2-amino 6-méthyl phénol ; le 5-acétamido 2-amino phénol et leurs sels d'addition avec un acide.

Parmi les bases hétérocycliques, on peut citer à titre d'exemple, les dérivés pyridiniques, les dérivés pyrimidiniques et les dérivés pyrazoliques. Parmi les dérivés pyridiniques, on peut citer les composés décrits par exemple dans les brevets GB 1 026 978 et GB 1 153 196, comme la 2,5-diamino pyridine ; la 2-(4-méthoxyphényl)amino 3-amino pyridine ; la 2,3-diamino 6-méthoxy pyridine ; la 2-(β-méthoxyéthylamino) 3-amino 6-méthoxy pyridine ; la 3,4-diamino pyridine et leurs sels d'addition avec un acide.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2 801 308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; la 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ainsi que leurs sels d'addition avec un acide ou avec une base.

Parmi les dérivés pyrimidiniques, on peut citer les composés décrits par exemple dans les brevets DE 23 59 399 ; JP 88-169571 ; JP 05-63124 ; EP 0 770 375 ou la demande de brevet WO 96/15765 comme la 2,4,5,6-tétra-aminopyrimidine ; la 4-hydroxy 2,5,6-triaminopyrimidine ; la 2-hydroxy 4,5,6-triaminopyrimidine ; la 2,4-dihydroxy

5,6-diaminopyrimidine ; la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol ; le 2-(7-amino pyrazolo-[1,5-a]-pyrimidin-3-ylamino)-éthanol ; le 2-[(3-amino-pyrazolo[1,5-a]pyrimidin-7-yl)-(2-hydroxy-éthyl)-amino]-éthanol ; le 2-[(7-amino-pyrazolo[1,5-a]pyrimidin-3-yl)-(2-hydroxy-éthyl)-amino]-éthanol ; la 5,6-diméthyl pyrazolo-[1,5-a]-pyrimidine-3,7-diamine ; la 2,6-diméthyl pyrazolo-[1,5-a]-pyrimidine-3,7-diamine ; la 2, 5, N 7, N 7-tetraméthyl pyrazolo-[1,5-a]-pyrimidine-3,7-diamine ; la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 38 43 892, DE 41 33 957 et les demandes de brevet WO 94/08969, WO 94/08970, FR-A-2 733 749 et DE 195 43 988 comme le 4,5-diamino 1-méthyl pyrazole ; le 4,5-diamino 1-(β-hydroxyéthyl) pyrazole ; le 3,4-diamino pyrazole ; le 4,5-diamino 1-(4'-chlorobenzyl) pyrazole ; le 4,5-diamino 1,3-diméthyl pyrazole ; le 4,5-diamino 3-méthyl 1-phényl pyrazole ; le 4,5-diamino 1-méthyl 3-phényl pyrazole ; le 4-amino 1,3-diméthyl 5-hydrazino pyrazole ; le 1-benzyl 4,5-diamino 3-méthyl pyrazole ; le 4,5-diamino 3-tert-butyl 1-méthyl pyrazole ; le 4,5-diamino 1-tert-butyl 3-méthyl pyrazole ; le 4,5-diamino 1-(β-hydroxyéthyl) 3-méthyl pyrazole ; le 4,5-diamino 1-éthyl 3-méthyl pyrazole ; le 4,5-diamino 1-éthyl 3-(4'-méthoxyphényl) pyrazole ; le 4,5-diamino 1-éthyl 3-hydroxyméthyl pyrazole ; le 4,5-diamino 3-hydroxyméthyl 1-méthyl pyrazole ; le 4,5-diamino 3-hydroxyméthyl 1-isopropyl pyrazole ; le 4,5-diamino 3-méthyl 1-isopropyl pyrazole ; le 4-amino 5-(2'-aminoéthyl)amino 1,3-diméthyl pyrazole ; le 3,4,5-triamino pyrazole ; le 1-méthyl 3,4,5-triamino pyrazole ; le 3,5-diamino 1-méthyl 4-méthylamino pyrazole ; le 3,5-diamino 4-(β-hydroxyéthyl)amino 1-méthyl pyrazole et leurs sels d'addition avec un acide.

En présence de bases d'oxydation additionnelles dans la composition de l'invention, la quantité de chacune d'elles est en générale comprise entre 0,001 et 10 % en poids environ du poids total de la composition tinctoriale, de préférence entre 0,005 et 6 %.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

La composition tinctoriale conforme à l'invention peut en outre contenir un ou plusieurs colorants directs pouvant notamment être choisis parmi les colorants nitrés de la série benzénique, les colorants directs azoïques, les colorants directs méthiniques. Ces colorants directs peuvent être de nature non ionique, anionique ou cationique.

Le milieu approprié pour la teinture appelé aussi support de teinture est un milieu cosmétique généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en $C_1$-$C_4$, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci-dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (III) suivante :

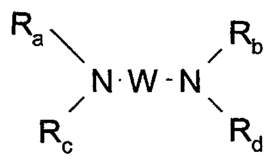
(III)

dans laquelle W est un reste propylène éventuellement substitué par un groupement hydroxyle ou un radical alkyle en $C_1$-$C_4$ ; $R_a$, $R_b$, $R_c$ et $R_d$, identiques ou différents, représentent un atome d'hydrogène, un radical alkyle en $C_1$-$C_4$ ou hydroxyalkyle en $C_1$-$C_4$.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Le procédé de la présente invention est un procédé dans lequel on applique sur les fibres la composition selon la présente invention telle que définie précédemment, et on révèle la couleur à l'aide d'un agent oxydant. La couleur peut être révélée à pH acide, neutre ou alcalin et l'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

L'invention a aussi pour objet un dispositif à plusieurs compartiments ou "kit" de teinture dans lequel un premier compartiment renferme la composition tinctoriale de la présente invention définie ci-dessus et un deuxième compartiment renferme un agent oxydant. Ce dispositif peut être équipé d'un moyen permettant de délivrer sur les cheveux le mélange souhaité, tel que les dispositifs décrits dans le brevet FR-2 586 913 au nom de la demanderesse.

A partir de ce dispositif, il est possible de teindre les fibres kératiniques à partir d'un procédé qui comprend le mélange d'une composition tinctoriale comprenant au moins un dérivé de triazacyclononane conforme à l'invention avec un agent oxydant, et l'application du mélange obtenu sur les fibres kératiniques pendant un temps suffisant pour développer la coloration désirée.

Les dérivés de triazacyclononane conforme à l'invention peuvent être obtenus par réaction nucléophile du cycle triazacyclononane sur une, deux ou trois molécules de para-fluoronitrobenzène éventuellement suivie d'une fonctionnalisation des atomes d'azote restants sur le cycle triazacyclononane et suivie ensuite d'une réduction des groupements nitro en amine. En particulier, ils peuvent être obtenus par analogie avec des procédés de préparation

décrits dans la littérature, voir notamment la demande de brevet DE 42 41 532. La réduction des groupements nitro en amine peut être réalisée en présence de zinc, voir notamment la référence J. heterocycl. Chem. 1992, 29 (6) 1513-1517.

L'invention a enfin pour objet les dérivés intermédiaires de triazacyclononane substitué sur l'un au moins des atomes d'azote par un groupement 4'-nitrophényle substitué ou non substitué.

Selon un mode de réalisation particulier de l'invention, les dérivés intermédiaires de triazacyclononane substitué sur l'un au moins des atomes d'azote par un groupement 4'-nitrophényle substitué ou non substitué sont choisis parmi les composés de formule (IV) suivante et leurs sels d'addition :

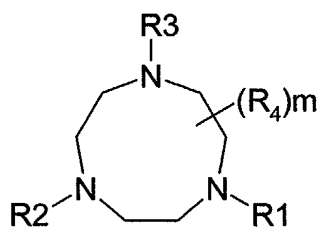

(IV)

dans laquelle :

- $R_1$, $R_2$ et $R_3$ représentent :
  - un atome hydrogène ;
  - un radical alkyle pouvant être saturé ou insaturé, non substitué ou substitué par un ou plusieurs radicaux carboxyle, alkylcarbonyle, alcoxycarbonyle, carbamoyle, mono- ou di-alkylcarbamoyle, alkylsulfonyle, trialkylammonium, N-alkylimidazolium, hétérocycliques à 4, 5, 6 ou 7 atomes, saturés ou insaturés, dont le ou les hétéroatomes sont choisis parmi l'azote, l'oxygène et le soufre ;
  - un radical $-CH_2-R'$ où R' est un radical alkyle pouvant être saturé ou insaturé, substitué par un ou plusieurs radicaux hydroxy, alcoxy, amino, mono- ou di-alkylamino, trialkylammonium, alkylcarbonylamino, alkylcarbonyloxy ;
  - un radical alkylcarbonyle pouvant être saturé ou insaturé, non substitué ou substitué par un ou plusieurs radicaux hydroxyle, alcoxy, amino, mono- ou di-alkylamino, alkylsulfonyle, carboxyle, alkylcarbonyle, alcoxycarbonyle, carbamoyle, mono- ou di-alkylcarbamoyle, trialkylammonium, N-alkylimidazolium, hétérocycliques à 4, 5, 6 ou 7 atomes, saturés ou insaturés, dont le ou les hétéroatomes sont choisis parmi l'azote, l'oxygène et le soufre ;
  - un radical alcoxycarbonyle ;
  - un radical mono- ou di-alkylcarbamoyle ;
  - un radical carbamoyle ;
  - un radical carboxyle ;
  - un radical alkylsulfonyle ;
  - un radical arylsulfonyle ;
  - un radical correspondant à la formule (V) :

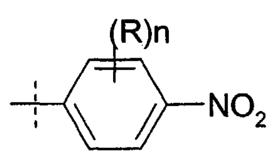

(V)

dans laquelle :

- R représente :
  - un radical alkyle pouvant être saturé ou insaturé, non substitué ou substitué par un ou plusieurs radicaux hydroxyle, amino, guanidinyle, trialkylammonium, N-alkylimidazolium, carboxyle, carbamoyle ;

- un radical alcoxy pouvant être saturé ou insaturé, non substitué ou substitué par un ou plusieurs radicaux hydroxyle, amino, trialkylammonium, N-alkylimidazolium ;
- un radical alcoxyalkyle ;
- un radical hydroxyalcoxyalkyle ;
- un radical aminoalkyle, l'amine pouvant être non substituée ou mono ou disubstituée par un radical alkyle, acétyle, hydroxyalkyle ;

• n est compris entre 0 et 4, étant entendu que lorsque n est supérieur à 1 alors les radicaux R peuvent être identiques ou différents ;

étant entendu qu'au moins un des radicaux $R_1$, $R_2$ ou $R_3$ est choisi parmi les radicaux de formule (II) ;

• $R_4$ représente :

- un radical alkyle pouvant être saturé ou insaturé ;
- un radical hydroxyalkyle ;
- un radical alcoxyalkyle ;
- un radical alkylcarbonyle ;
- un radical hydroxyalcoxyalkyle ;
- un radical aminoalkyle, l'amine pouvant être non substituée ou mono ou disubstituée par un radical alkyle, acétyle, hydroxyalkyle ;
- un radical hydroxy et aminoalkyle ;
- un radical carboxyle ;
- un radical carboxyalkyle ;
- un radical carbamoyle ;
- un radical carbamoylalkyle ;
- un radical alcoxycarbonyle ;
- un radical mono- ou di-alkylaminocarbonyle ;

• m est compris entre 0 et 6, étant entendu que lorsque m est supérieur à 1 alors les radicaux $R_4$ peuvent être identiques ou différents.

Les exemples qui suivent servent à illustrer l'invention sans toutefois présenter un caractère limitatif.

EXEMPLES

**Exemple 1 : synthèse du 1-(4-amino-phényl)-[1,4,7]triazonane (2)**

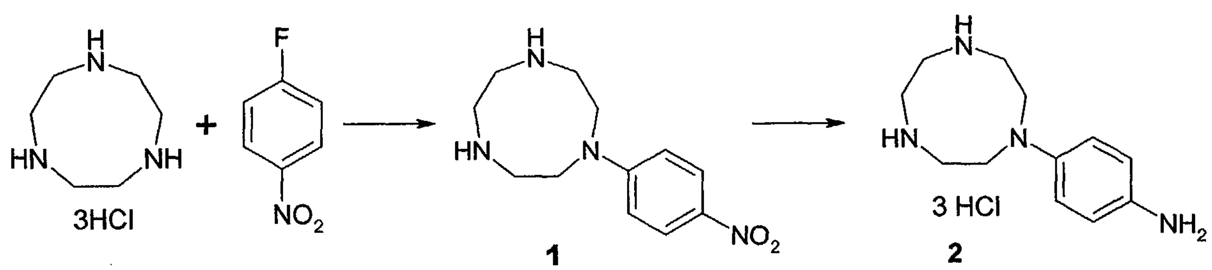

Préparation du 1-(4-nitro-phényl)-[1,4,7]triazonane (1)

1 g de triazacyclononane trichlorhydrate (4,2 mmol) et 0,4 g d'hydrure de sodium (0,01 mmol) sont placés dans un ballon contenant 15 ml de tétrahydrofurane anhydre. A ce mélange sont ajoutés 0,296 g de 1-fluoro-4-nitrobenzène (2,1 mmol) à température ambiante. Après 6 heures de réaction, l'excès d'hydrure de sodium est hydrolysé avec de l'eau et le THF est évaporé. La phase aqueuse est ensuite extraite avec du dichlorométhane. Les phases organiques sont réunies, séchées avec du sulfate de magnésium puis évaporées. Le produit brut obtenu est recristallisé dans du dichlorométhane pour donner 0,03 g d'un solide jaune, soit un rendement de 3 %. Les résultats d'analyses

obtenus par RMN $^1H$ et par spectrométrie de masse sont les suivants :
**$^1$H RMN** (400MHz, DMSO) : 2,72 (s, 4H) ; 3,1 (m, 4H) ; 3,76 (m, 4H) ; 6,5 (s, 2H) ; 6,85 (d, 2H) ; 8,09 (d, 2H).
**Masse** (ESI+) : 251 (MH+).

Préparation du 1-(4-amino-phényl)-[1,4,7]triazonane (2)

Le 1-(4-nitro-phényl)-[1,4,7]triazonane (1) précédemment obtenu est additionné dans 10 ml d'éthanol et réduit en présence de zinc et de chlorure d'ammonium. Le 1-(4-amino-phényl)-[1,4,7]triazonane (2) obtenu est isolé sous forme de chlorhydrate.

**Exemple 2: synthèse du 1,4-bis-(4-amino-phényl)-[1,4,7]triazonane (4)**

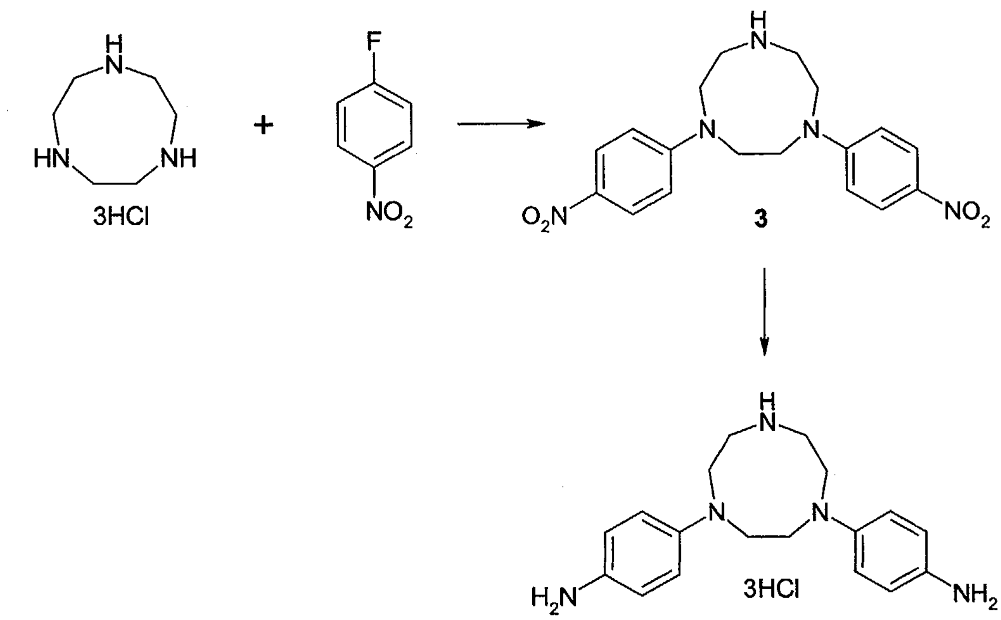

3

4

Préparation du 1,4-bis-(4-nitro-phényl)-[1,4,7]triazonane (3)

20 g de triazacyclononane trichlorhydrate (83,8 mmol), 35,4 g de 1-fluoro-4-nitro-benzène (251,4 mmol) et 40 g de carbonate de potassium (290 mmol) sont placés dans 150 ml d'eau et le mélange est porté à reflux sous agitation pendant 24 heures. Après refroidissement à température ambiante, le solide jaune orangé obtenu est filtré et rincé à l'eau puis à l'éther de pétrole. Après séchage, 31 g de produit sont obtenus, soit un rendement de 99 %.
Les résultats d'analyses obtenus par RMN $^1H$ et par spectrométrie de masse sont les suivants :
**$^1$H RMN** (400MHz, DMSO) : 2,2 (m, 1H) ; 2,8 (m, 4H) ; 3,4 (m, 4H) ; 3,9 (m, 4H) ; 6,8 (m, 4H) ; 8,1 (m, 4H).
**Masse** (ESI+): m/z = 372 (MH+).

Préparation du 1,4-bis-(4-amino-phényl)-[1,4,7]triazonane chlorhydrate (4)

2 g de 1,4-bis-(4-nitro-phényl)-[1,4,7]triazonane (3) précédemment obtenu (5,3 mmol) et 0,5 g de palladium sur charbon en suspension dans 450 ml d'éthanol sont placés dans un hydrogénateur. L'hydrogénation est réalisée à 80 °C sous 10 bars de pression d'hydrogène. Après refroidissement à température ambiante et filtration du catalyseur, 2 g de produit sont isolés sous forme de chlorhydrate, soit un rendement de 80 %.
Les résultats d'analyses obtenus par RMN $^1H$ et par spectrométrie de masse sont les suivants :
**$^1$H RMN** (400MHz, $D_2O$) : 3,3 (m, 4H) ; 3,7 (m, 8H) ; 6,9 (m, 4H) ; 7,3 (m, 4H).
**Masse** (ESI+) : m/z = 312 (MH+).

**Exemple 3 : synthèse du 1,4,7-tris-(4-amino-phényl)-[1,4,7]triazacyclononane (6)**

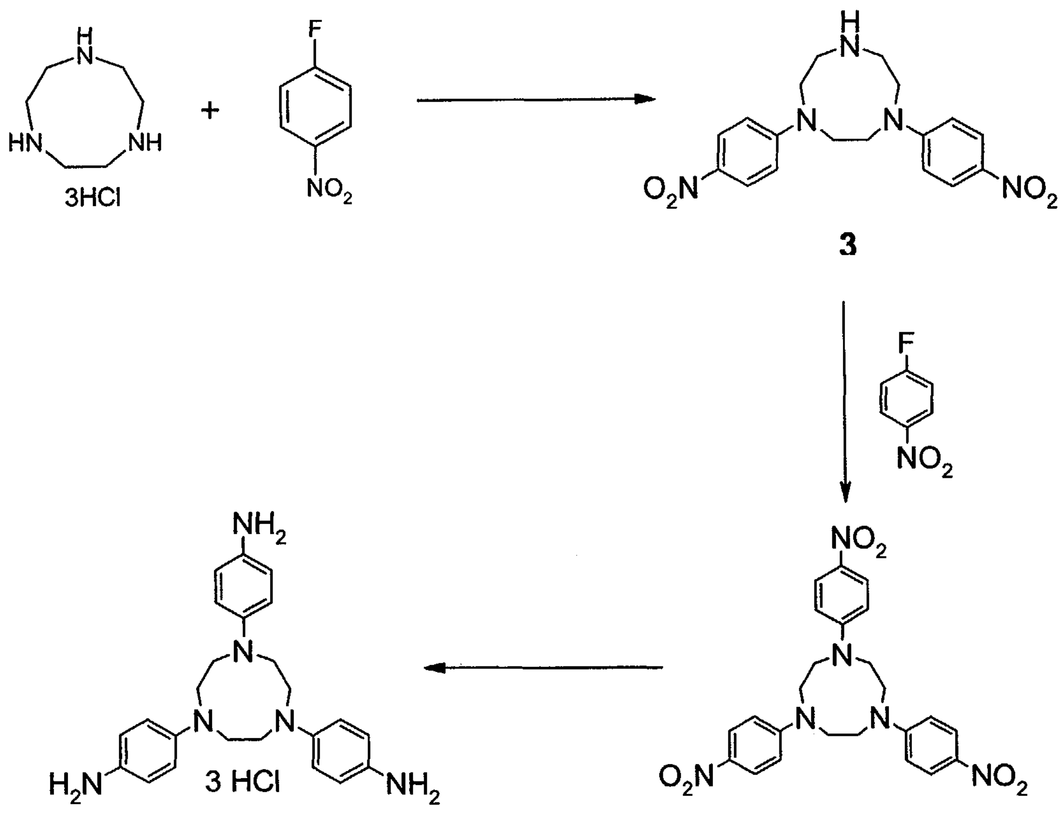

3

6

5

Préparation du 1,4,7-tris-(4-nitro-phényl)-[1,4,7]triazacyclononane (5)

5 g de 1,4-bis-(4-nitro-phényl)-[1,4,7]triazonane (3) obtenu précédemment (0,013 mol), 2,28 g de para-fluoro-nitro-benzène (0,016 mol) et 2,24 g de carbonate de potassium (0,016 mol) sont placés dans 25 ml de N-méthyl pyrrolidinone et le mélange est porté à 120 °C pendant 13 heures. Le milieu réactionnel est versé dans une solution aqueuse saturée de chlorure de sodium pour donner un précipité jaune. 0,3 g de produit est obtenu par purification par chromatographie, soit un rendement de 5 %.
Les résultats d'analyses obtenus par RMN $^1H$ et par spectrométrie de masse sont les suivants :
**$^1$H RMN** (400MHz, DMSO) : 3,75 (s, 12H) ; 6,76 (m, 6H) ; 8 (m, 6H).
**Masse** (ionisation chimique $NH_3$) : 493 ($MH^+$) et 510 ($MNH_4^+$).

Préparation du 1,4,7-tris-(4-amino-phényl)-[1,4,7]triazonane (6)

Le 1,4,7-tris-(4-nitro-phényl)-[1,4,7]triazacyclononane (5) précédemment obtenu est additionné dans 300 ml d'éthanol et réduit en présence de zinc et de chlorure d'ammonium. Le 1,4,7-tris-(4-amino-phényl)-[1,4,7]triazonane (6) obtenu est isolé sous forme de chlorhydrate.

**Exemple 4: synthèse du 2-[4,7-bis-(4-amino-phényl)-[1,4,7]triazonan-1-yl]éthanol (8)**

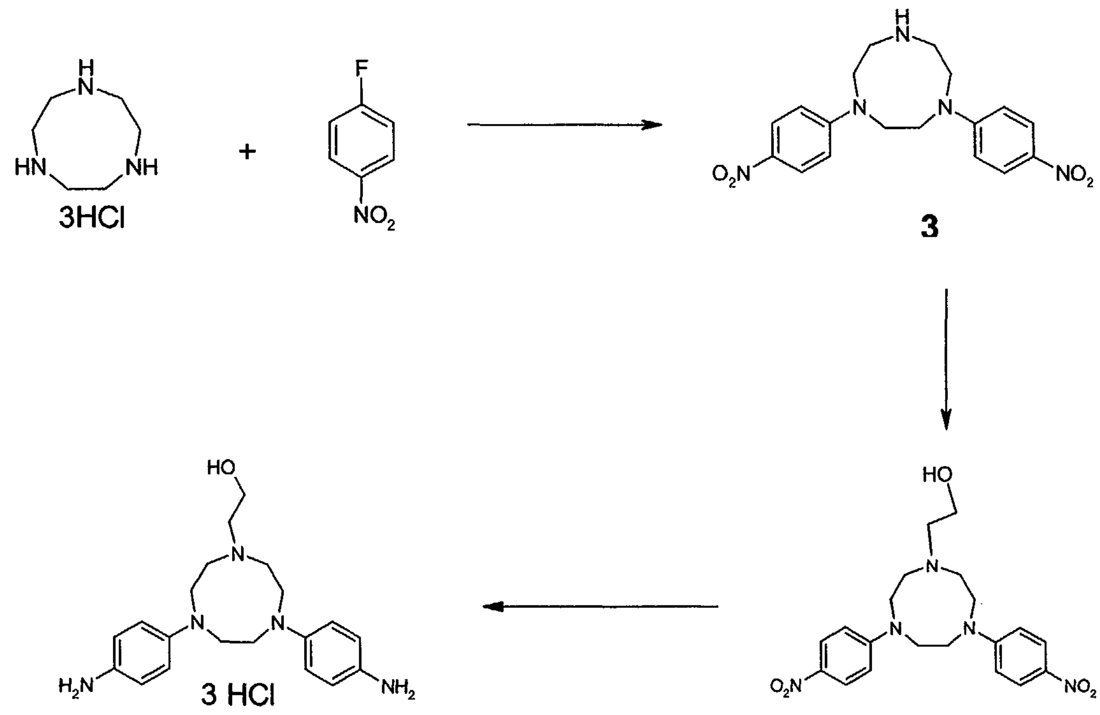

3

8

7

Préparation du 2-[4,7-bis-(4-nitro-phényl)-[1,4,7]triazonan-1-yl]éthanol (7)

5 g de 1,4-bis-(4-nitro-phényl)-[1,4,7]triazonane (3) obtenu précédemment (13,46 mmol) et 2,8 ml de triéthylamine (20,2 mmol) sont placés dans 13 ml de NMP. A ce mélange sont ajoutés 1,43 ml de bromoéthanol (20,2 mmol). Le milieu réactionnel ainsi obtenu est chauffé à 110 °C pendant 7 heures. De l'eau est ajoutée sous agitation, et le précipité formé est filtré puis rincé abondamment à l'eau, puis à l'éther de diisopropyle. Après séchage, 5,6 g de produit sont obtenus, soit un rendement d'environ 100 %.
Les résultats d'analyses obtenus par RMN $^1$H et par spectrométrie de masse sont les suivants :
**RMN $^1$H** (DMSO, 400MHz) : 2,59 (t, 2H) ; 2,7 (m, 4H) ; 3,29 (q + s, 2H + $H_2O$) ; 3,46 (m, 4H) ; 3,85 (s, 4H) ; 4,33 (t, 1H) ; 6,8( m, 4H) ; 8.04 (m, 4H).
**Masse** ( ESI+/- dans MeOH/$H_2O$) : m/z = 416 ( M+H)+ ; 414 (M-H)-.

Préparation du 2-[4,7-bis-(4-amino-phényl)-[1,4,7]triazonan-1-yl]éthanol (8)

Dans un hydrogénateur, 5 g de 2-[4,7-bis-(4-nitro-phényl)-[1,4,7]triazonan-1-yl]-éthanol (7) obtenu précédemment (5,3 mmol) sont placés dans 50 ml d'acide acétique et 100 ml d'éthanol en présence de 1 g de palladium sur charbon à 5 %. L'hydrogénation est réalisée à 80 °C sous une pression de 10 bars d'hydrogène pendant 4 heures. Après refroidissement à température ambiante et filtration du catalyseur, 2,4 g de produit est isolé sous forme de chlorhydrate, soit un rendement de 43 %.
Les résultats d'analyses obtenus par RMN $^1$H et par spectrométrie de masse sont les suivants :
**RMN $^1$H** (400MHz, D2O) : 3,49 (t, 2H) ; 3,62-3,94 (t + m, 14H) ; 7,05-7,35 (2d, 8H).
**Masse** (ESI+) : m/z = 356 (MH+).

**Exemple 5 : synthèse de le chlorure de 3-{3-[4,7-bis-(4-amino-phényl)-[1,4,7] triazonan-1-yl]-propyl}-1-méthyl-3H-imidazol-1-ium (10)**

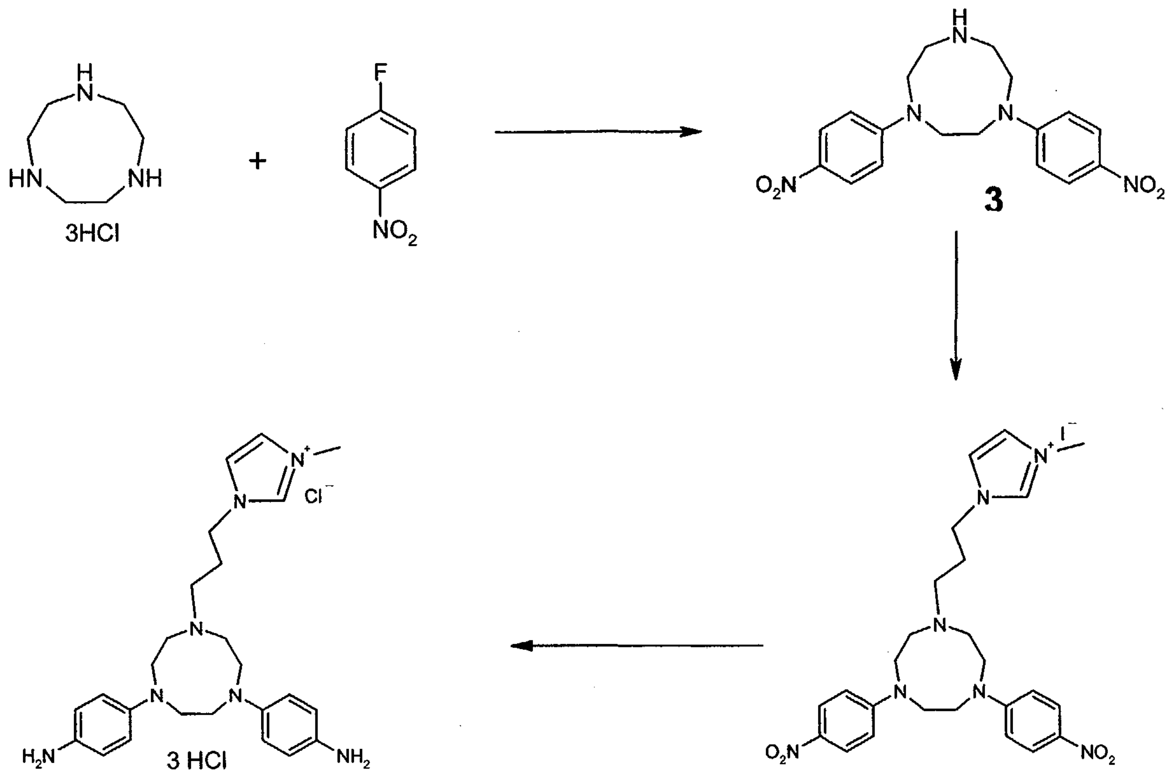


**10** **9**

**10 9**

Préparation du 3-{3-[4,7-bis-(4-nitro-phényl)-[1,4,7] triazonan-1-yl]-propyl}-1-methyl-3H-imidazol-1-ium (9)

Dans un tricol de 25 ml, sont introduits 0,5 g de 1,4-bis-(4-nitro-phényl)-[1,4,7]triazonane (3) obtenu précédemment (1,35 mmol), puis 5 ml de NMP, 0,19 ml de triéthylamine (1,35 mmol) puis enfin le dérivé iodé 3-(3-iodo-propyl)-1-methyl-3H-imidazol-1-ium. Le milieu réactionnel est porté à 120 °C pendant 17 heures. Le milieu réactionnel refroidi est versé à température ambiante dans une solution de saumur. Un solide gommeux jaune est isolé, lavé à l'acétate d'éthyle puis à l'eau pour donner une poudre jaune.
Les résultats d'analyses obtenus par RMN $^1$H et par spectrométrie de masse sont les suivants :
**RMN $^1$H** (DMSO, 400MHz) : 1,75 ( m, 2H) ; 2,5 ( t, 2H + DMSO) ; 2,68 (m, 4H) ; 3,48 ( m, 4H) ; 3,83 (s, 3H) ; 3,84 (s, 4H) ; 4,01 (m, 2H) ; 6,81 (d, 4H) ; 7,63 (dd, 1H) ; 7,67 (dd, 1H) ; 8,05 (d, 4H) ; 9,02 (s, 1H).
**Masse** (ESI+/- dans MeOH/$H_2O$) : m/z = 494 (M)+ ; 127 (I-).

**Préparation du 3-{3-[4,7-bis-(4-amino-phényl)-[1,4,7]triazonan-1-yl]-propyl}-1-methyl-3H-imidazol-1-ium (10)**

Le 3-{3-[4,7-bis-(4-nitro-phényl)-[1,4,7]triazonan-1-yl]-propyl}-1-methyl-3H-imidazol-1-ium (9) précédemment obtenu est additionné dans 50 ml d'éthanol et réduit en présence de zinc et de chlorure d'ammonium. Le 3-{3-[4,7-bis-(4-amino-phényl)-[1,4,7]triazonan-1-yl]-propyl}-1-methyl-3H-imidazol-1-ium (10) obtenu est isolé sous forme de chlorhydrate.

**EXEMPLES DE TEINTURE**

**Exemples 1-14 : compositions tinctoriales à partir du 1-(4-amino-phényl)-[1,4,7]triazonane (2).**

Exemples 1 à 7 : teinture en milieu acide

Les compositions tinctoriales suivantes sont préparées :

| Exemple | 1 | 2 | 3 | 4 | 5 | 6 | 7 |
|---|---|---|---|---|---|---|---|
| 1-(4-amino-phényl) [1,4,7] triazonane | $4.10^{-4}$ mole | $4.10^{-4}$ mole | $4.10^{-4}$ mole | $4.10^{-4}$ mole | $4.10^{-4}$ mole | $4.10^{-4}$ mole | $4.10^{-4}$ mole |
| Benzène-1,3-diol | $4.10^{-4}$ mole | - | - | - | - | - | - |
| 5-amino 2-méthyl phénol | | $4.10^{-4}$ mole | - | - | - | - | - |
| 1H-indol-6-ol | - | - | $4.10^{-4}$ mole | - | - | - | - |
| 2-amino pyridin-3-ol | - | - | - | $4.10^{-4}$ mole | - | - | - |
| 3,6-diméthyl-1 H-pyrazolo [5,1-c] [1,2,4] triazole | - | - | - | - | $4.10^{-4}$ mole | - | - |
| 2-(2,4-diamino-phénoxy)-éthanol ; chlorhydrate | - | - | - | - | - | $4.10^{-4}$ mole | - |
| 3-amino 2-chloro 6-méthyl phénol ; chlorhydrate | | - | - | - | - | - | $4.10^{-4}$ mole |
| Support de teinture | (1) | (1) | (1) | (1) | (1) | (1) | (1) |
| Eau déminéralisée q.s.p. | 100 g | 100 g | 100 g | 100 g | 100 g | 100 g | 100 g |

(1) : Support de teinture pH 7

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en $C_8$-$C_{10}$ polyglucoside en solution aqueuse à 60% | 3,6 g M.A |
| Alcool benzylique | 2,0 g |
| Polyéthylène glycol à 8 motifs d'oxyde d'éthylène | 3,0 g |
| $Na_2HPO_4$ | 0,28 g |
| $KH_2PO_4$ | 0,46 g |

Au moment de l'emploi, chaque composition est mélangée avec un poids égal d'eau oxygénée à 20 volumes (6 % en poids). On obtient un pH final de 7.

Chaque mélange obtenu est appliqué sur des mèches de cheveux gris à 90 % de blancs. Après 30 minutes de pose, les mèches sont rinçées, lavées avec un shampooing standard, rinçées à nouveau puis séchées.

| **Exemple** | **1** | **2** | **3** | **4** | **5** | **6** | **7** |
|---|---|---|---|---|---|---|---|
| **Nuance observée** | Brun jaune | Brun | Brun | Brun | Brun jaune | Gris bleu vert | Gris violet |

Exemples 8 à 14 : teinture en milieu basique

Les compositions tinctoriales suivantes sont préparées :

| **Exemple** | **8** | **9** | **10** | **11** | **12** | **13** | **14** |
|---|---|---|---|---|---|---|---|
| 1-(4-amino-phényl) [1,4,7] triazonane | $4.10^{-4}$ mole | $4.10^{-4}$ mole | $4.10^{-4}$ mole | $4.10^{-4}$ mole | $4.10^{-4}$ mole | $4.10^{-4}$ mole | $4.10^{-4}$ mole |
| Benzène-1,3-diol | $4.10^{-4}$ mole | - | - | - | - | - | - |
| 5-amino 2-méthyl phénol | | $4.10^{-4}$ mole | - | - | - | - | - |
| 1 -indol-6-ol | - | - | $4.10^{-4}$ mole | - | - | - | - |
| 2-amino pyridin-3-ol | - | - | - | $4.10^{-4}$ mole | - | - | - |
| 3,6-diméthyl-1H-pyrazolo [5,1-c] [1,2,4] triazole | - | - | - | - | $4.10^{-4}$ mole | - | - |
| 2-(2,4-diamino-phénoxy)-éthanol ; chlorhydrate | - | - | - | - | - | $4.10^{-4}$ mole | - |
| 3-amino 2-chloro 6-méthyl phénol ; chlorhydrate | - | - | - | - | - | - | $4.10^{-4}$ mole |
| Support de teinture | (2) | (2) | (2) | (2) | (2) | (2) | (2) |
| Eau déminéralisée q.s.p. | 100 g | 100 g | 100 g | 100 g | 100 g | 100 g | 100 g |

(2) : Support de teinture pH 9,5

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en $C_8$-$C_{10}$ polyglucoside en solution aqueuse à 60% | 3,6 g M.A |
| Alcool benzylique | 2,0 g |
| Polyéthylène glycol à 8 motifs d'oxyde d'éthylène | 3,0 g |
| $NH_4Cl$ | 4,32 g |

(suite)

| | |
|---|---|
| Ammoniaque à 20% de $NH_3$ | 2,94 g |

Au moment de l'emploi, chaque composition est mélangée avec un poids égal d'eau oxygénée à 20 volumes (6 % en poids). On obtient un pH final de 9,5.

Chaque mélange obtenu est appliqué sur des mèches de cheveux gris à 90 % de blancs. Après 30 minutes de pose, les mèches sont rinçées, lavées avec un shampooing standard, rinçées à nouveau puis séchées.

| **Exemple** | **8** | **9** | **10** | **11** | **12** | **13** | **14** |
|---|---|---|---|---|---|---|---|
| **Nuance observée** | Jaune orangé | Brun | Orange | Orange | Rouge | Gris bleu | Gris violet |

**Exemples 15 à 28 : compositions tinctoriales à partir du 1,4-bis-(4-amino-phényl)-[1,4,7]triazonane (4)**

Exemples 15 à 21 : teinture en milieu acide

Les compositions tinctoriales suivantes sont préparées :

| **Exemple** | **15** | **16** | **17** | **18** | **19** | **20** | **21** |
|---|---|---|---|---|---|---|---|
| 1,4-bis-(4-amino-phényl)-[1,4,7] triazonane | $4.10^{-4}$ mole | $4.10^{-4}$ mole | $4.10^{-4}$ mole | $4.10^{-4}$ mole | $4.10^{-4}$ mole | $4.10^{-4}$ mole | $4.10^{-4}$ mole |
| Benzène-1,3-diol | $4.10^{-4}$ mole | - | - | - | - | - | - |
| 5-amino 2-méthyl phénol | - | $4.10^{-4}$ mole | - | - | - | - | - |
| 1H-indol-6-ol | - | - | $4.10^{-4}$ mole | - | - | - | - |
| 2-amino pyridin-3-ol | - | - | - | $4.10^{-4}$ mole | - | - | - |
| 3,6-diméthyl-1H-pyrazolo[5,1-c][1,2,4]triazole | - | - | - | - | $4.10^{-4}$ mole | - | - |
| 2-(2,4-diamino-phénoxy)-éthanol ; chlorhydrate | - | - | - | - | - | $4.10^{-4}$ mole | - |
| 3-amino 2-chloro 6-méthyl phénol ; chlorhydrate | - | - | - | - | - | - | $4.10^{-4}$ mole |
| Support de teinture | (1) | (1) | (1) | (1) | (1) | (1) | (1) |
| Eau déminéralisée q.s.p. | 100 g | 100 g | 100 g | 100 g | 100 g | 100 g | 100 g |

(1) : Support de teinture pH 7

| | |
|---|---|
| Alcool éthylique à 96° | 20,8 g |
| Métabisulfite de sodium en solution aqueuse à 35% | 0,23 g M.A |

(suite)

| | |
|---|---|
| Sel pentasodique de l'acide diéthylène-triamine-pentaacétique en solution aqueuse à 40% | 0,48 g M.A |
| Alkyl en $C_8$-$C_{10}$ polyglucoside en solution aqueuse à 60% | 3,6 g M.A |
| Alcool benzylique | 2,0 g |
| Polyéthylène glycol à 8 motifs d'oxyde d'éthylène | 3,0 g |
| $Na_2HPO_4$ | 0,28 g |
| $KH_2PO_4$ | 0,46 g |

Au moment de l'emploi, chaque composition est mélangée avec un poids égal d'eau oxygénée à 20 volumes (6 % en poids). On obtient un pH final de 7.

Chaque mélange obtenu est appliqué sur des mèches de cheveux gris à 90 % de blancs. Après 30 minutes de pose, les mèches sont rinçées, lavées avec un shampooing standard, rinçées à nouveau puis séchées.

| **Exemple** | **15** | **16** | **17** | **18** | **19** | **20** | **21** |
|---|---|---|---|---|---|---|---|
| **Nuance observée** | Gris violet | Gris violet | Gris violet | Gris violet | Brun | Gris violet | Gris violet |

<u>Exemples 22 à 28 : teinture en milieu basique</u>

Les compositions tinctoriales suivantes sont préparées :

| **Exemple** | **22** | **23** | **24** | **25** | **26** | **27** | **28** |
|---|---|---|---|---|---|---|---|
| 1,4-bis-4-amino-phényl)-[1,4,7] triazonane | $4.10^{-4}$ mole | $4.10^{-4}$ mole | $4.10^{-4}$ mole | $4.10^{-4}$ mole | $4.10^{-4}$ mole | $4.10^{-4}$ mole | $4.10^{-4}$ mole |
| Benzène-1,3-diol | $4.10^{-4}$ mole | - | - | - | - | - | - |
| 5-amino 2-méthyl phénol | - | $4.10^{-4}$ mole | - | - | - | - | - |
| 1H-indol-6-ol | - | - | $4.10^{-4}$ mole | - | - | - | - |
| 2-amino pyridin-3-ol | - | - | - | $4.10^{-4}$ mole | - | - | - |
| 3,6-diméthyl-1H-pyrazolo [5,1-c] [1,2,4] triazole | - | - | - | - | $4.10^{-4}$ mole | - | - |
| 2-(2,4-diamino-phénoxy)-éthanol ; chlorhydrate | - | - | - | - | - | $4.10^{-4}$ mole | - |
| 3-amino 2-chloro 6-méthyl phénol ; chlorhydrate | - | - | - | - | - | - | $4.10^{-4}$ mole |
| Support de teinture | (2) | (2) | (2) | (2) | (2) | (2) | (2) |

(2) : Support de teinture pH 9,5

(suite)

| Exemple | 22 | 23 | 24 | 25 | 26 | 27 | 28 |
|---|---|---|---|---|---|---|---|
| Eau déminéralisée q.s.p. | 100 g | 100 g | 100 g | 100 g | 100 g | 100 g | 100 g |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en $C_8$-$C_{10}$ polyglucoside en solution aqueuse à 60% | 3,6 g M.A |
| Alcool benzylique | 2,0 g |
| Polyéthylène glycol à 8 motifs d'oxyde d'éthylène | 3,0 g |
| $NH_4Cl$ | 4,32 g |
| Ammoniaque à 20% de $NH_3$ | 2,94 g |

Au moment de l'emploi, chaque composition est mélangée avec un poids égal d'eau oxygénée à 20 volumes (6 % en poids). On obtient un pH final de 9,5.

Chaque mélange obtenu est appliqué sur des mèches de cheveux gris à 90 % de blancs. Après 30 minutes de pose, les mèches sont rinçées, lavées avec un shampooing standard, rinçées à nouveau puis séchées.

| Exemple | 22 | 23 | 24 | 25 | 26 | 27 | 28 |
|---|---|---|---|---|---|---|---|
| **Nuance observée** | Brun | Violet | Rouge | Brun | Violet | Violet | Violet |

**Exemples 29 à 42 : compositions tinctoriales à partir du 1,4,7-tris-(4-amino-phényl)-[1,4,7]triazonane (6)**

Exemples 29 à 35 : teinture en milieu acide

Les compositions tinctoriales suivantes sont préparées :

| Exemple | 29 | 30 | 31 | 32 | 33 | 34 | 35 |
|---|---|---|---|---|---|---|---|
| 1,4,7-tris-(4-amino-phényl)-[1,4,7] triazonane | $4.10^{-4}$ mole | $4.10^{-4}$ mole | $4.10^{-4}$ mole | $4.10^{-4}$ mole | $4.10^{-4}$ mole | $4.10^{-4}$ mole | $4.10^{-4}$ mole |
| Benzène-1,3-diol | $4.10^{-4}$ mole | - | - | - | - | - | - |
| 5-amino 2-méthyl phénol | - | $4.10^{-4}$ mole | - | - | - | - | - |
| 1H-indol-6-ol | - | - | $4.10^{-4}$ mole | - | - | - | - |
| 2-amino pyridin-3-ol | - | - | - | $4.10^{-4}$ mole | - | - | - |
| 3,6-diméthyl-1H-pyrazolo[5,1-c][1,2,4]triazole | - | - | - | - | $4.10^{-4}$ mole | - | - |
| 2-(2,4-diamino-phénoxy)-éthanol ; chlorhydrate | - | - | - | - | - | $4.10^{-4}$ mole | - |

(suite)

| Exemple | 29 | 30 | 31 | 32 | 33 | 34 | 35 |
|---|---|---|---|---|---|---|---|
| 3-amino 2-chloro 6-méthyl phénol ; chlorhydrate | - | - | - | - | - | - | $4.10^{-4}$ mole |
| Support de teinture | (1) | (1) | (1) | (1) | (1) | (1) | (1) |
| Eau déminéralisée q.s.p. | 100 g | 100 g | 100 g | 100 g | 100 g | 100 g | 100 g |

(1) : Support de teinture pH 7

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en $C_8$-$C_{10}$ polyglucoside en solution aqueuse à 60% | 3,6 g M.A |
| Alcool benzylique | 2,0 g |
| Polyéthylène glycol à 8 motifs d'oxyde d'éthylène | 3,0 g |
| $Na_2HPO_4$ | 0,28 g |
| $KH_2PO_4$ | 0,46 g |

Au moment de l'emploi, chaque composition est mélangée avec un poids égal d'eau oxygénée à 20 volumes (6 % en poids). On obtient un pH final de 7.

Chaque mélange obtenu est appliqué sur des mèches de cheveux gris à 90 % de blancs. Après 30 minutes de pose, les mèches sont rinçées, lavées avec un shampooing standard, rinçées à nouveau puis séchées.

| Exemple | 29 | 30 | 31 | 32 | 33 | 34 | 35 |
|---|---|---|---|---|---|---|---|
| **Nuance observée** | Jaune | Jaune orangé | Brun jaune | Jaune | Jaune | Gris bleu vert | Brun jaune |

Exemples 36 à 42 : teintures en milieu basique

Les compositions tinctoriales suivantes sont préparées :

| Exemple | 36 | 37 | 38 | 39 | 40 | 41 | 42 |
|---|---|---|---|---|---|---|---|
| 1,4,7-tris-(4-amino-phényl)-[1,4,7] triazonane | $4.10^{-4}$ mole | $4.10^{-4}$ mole | $4.10^{-4}$ mole | $4.10^{-4}$ mole | $4.10^{-4}$ mole | $4.10^{-4}$ mole | $4.10^{-4}$ mole |
| Benzène-1,3-diol | $4.10^{-4}$ mole | - | - | - | - | - | - |
| 5-amino 2-méthyl phénol | - | $4.10^{-4}$ mole | - | - | - | - | - |
| 1H-indol-6-ol | - | - | $4.10^{-4}$ mole | - | - | - | - |
| 2-amino pyridin-3-ol | - | - | - | $4.10^{-4}$ mole | - | - | - |
| 3,6-diméthyl-1H-pyrazolo[5,1-c] [1,2,4]triazole | - | - | - | - | $4.10^{-4}$ mole | - | - |

(suite)

| Exemple | 36 | 37 | 38 | 39 | 40 | 41 | 42 |
|---|---|---|---|---|---|---|---|
| 2-(2,4-diamino-phénoxy)-éthanol ; chlorhydrate | - | - | - | - | - | $4.10^{-4}$ mole | - |
| 3-amino 2-chloro 6-méthyl phénol ; chlorhydrate | - | - | - | - | - | - | $4.10^{-4}$ mole |
| Support de teinture | (2) | (2) | (2) | (2) | (2) | (2) | (2) |
| Eau déminéralisée q.s.p. | 100 g | 100 g | 100 g | 100 g | 100 g | 100 g | 100 g |

(2) : Support de teinture pH 9,5

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en $C_8$-$C_{10}$ polyglucoside en solution aqueuse à 60% | 3,6 g M.A |
| Alcool benzylique | 2,0 g |
| Polyéthylène glycol à 8 motifs d'oxyde d'éthylène | 3,0 g |
| $NH_4Cl$ | 4,32 g |
| Ammoniaque à 20% de $NH_3$ | 2,94 g |

Au moment de l'emploi, chaque composition est mélangée avec un poids égal d'eau oxygénée à 20 volumes (6 % en poids). On obtient un pH final de 9,5.

Chaque mélange obtenu est appliqué sur des mèches de cheveux gris à 90 % de blancs. Après 30 minutes de pose, les mèches sont rinçées, lavées avec un shampooing standard, rinçées à nouveau puis séchées.

| Exemple | 36 | 37 | 38 | 39 | 40 | 41 | 42 |
|---|---|---|---|---|---|---|---|
| **Nuance observée** | Brun orangé | Jaune Orangé | Jaune orangé | Rouge | Gris bleu vert | Brun | Brun |

**Exemples 43 à 56 : compositions tinctoriales à partir du 2-[4,7-bis-(4-amino-phényl)-[1,4,7] triazonan-1-yl]éthanol (8)**

Exemples 43 à 49 : teinture en milieu acide

Les compositions tinctoriales suivantes sont préparées :

| Exemple | 43 | 44 | 45 | 46 | 47 | 48 | 49 |
|---|---|---|---|---|---|---|---|
| 2-[4,7-bis-(4-amino-phényl)-[1,4,7] triazonan-1-yl] éthanol | $4.10^{-4}$ mole | $4.10^{-4}$ mole | $4.10^{-4}$ mole | $4.10^{-4}$ mole | $4.10^{-4}$ mole | $4.10^{-4}$ mole | $4.10^{-4}$ mole |
| Benzène-1,3-diol | $4.10^{-4}$ mole | - | - | - | - | - | - |
| 5-amino 2-méthyl phénol | - | $4.10^{-4}$ mole | - | - | - | - | - |
| 1H-indol-6-ol | - | - | $4.10^{-4}$ mole | - | - | - | - |

(suite)

| Exemple | 43 | 44 | 45 | 46 | 47 | 48 | 49 |
|---|---|---|---|---|---|---|---|
| 2-amino pyridin-3-ol | - | - | - | $4.10^{-4}$ mole | - | - | - |
| 3,6-diméthyl-1H-pyrazolo[5,1-c][1,2,4]triazole | - | - | - | - | $4.10^{-4}$ mole | - | - |
| 2-(2,4-diamino-phénoxy)-éthanol ; chlorhydrate | - | - | - | - | - | $4.10^{-4}$ mole | - |
| 3-amino 2-chloro 6-méthyl phénol ; chlorhydrate | - | - | - | - | - | - | $4.10^{-4}$ mole |
| Support de teinture | (1) | (1) | (1) | (1) | (1) | (1) | (1) |
| Eau déminéralisée q.s.p. | 100 g | 100 g | 100 g | 100 g | 100 g | 100 g | 100 g |

(1) : Support de teinture pH 7

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en $C_8$-$C_{10}$ polyglucoside en solution aqueuse à 60% | 3,6 g M.A |
| Alcool benzylique | 2,0 g |
| Polyéthylène glycol à 8 motifs d'oxyde d'éthylène | 3,0 g |
| $Na_2HPO_4$ | 0,28 g |
| $KH_2PO_4$ | 0,46 g |

Au moment de l'emploi, chaque composition est mélangée avec un poids égal d'eau oxygénée à 20 volumes (6 % en poids). On obtient un pH final de 7.

Chaque mélange obtenu est appliqué sur des mèches de cheveux gris à 90 % de blancs. Après 30 minutes de pose, les mèches sont rinçées, lavées avec un shampooing standard, rinçées à nouveau puis séchées.

| Exemple | 43 | 44 | 45 | 46 | 47 | 48 | 49 |
|---|---|---|---|---|---|---|---|
| **Nuance observée** | Brun rouge intense | Gris rouge intense | Gris rouge intense | Gris intense | Brun rouge intense | Bleu intense | Gris violet intense |

Exemples 50 à 56 : teintures en milieu basique

Les compositions tinctoriales suivantes sont préparées :

| Exemple | 50 | 51 | 52 | 53 | 54 | 55 | 56 |
|---|---|---|---|---|---|---|---|
| 2-[4,7-bis-(4-amino-phényl)-[1,4,7]triazonan-1-yl] éthanol | $4.10^{-4}$ mole | $4.10^{-4}$ mole | $4.10^{-4}$ mole | $4.10^{-4}$ mole | $4.10^{-4}$ mole | $4.10^{-4}$ mole | $4.10^{-4}$ mole |
| Benzène-1,3-diol | $4.10^{-4}$ mole | - | - | - | - | - | - |

(suite)

| Exemple | 50 | 51 | 52 | 53 | 54 | 55 | 56 |
|---|---|---|---|---|---|---|---|
| 5-amino 2-méthyl phénol | - | $4.10^{-4}$ mole | - | - | - | - | - |
| 1H-indol-6-ol | - | - | $4.10^{-4}$ mole | - | - | - | - |
| 2-amino pyridin-3-ol | - | - | - | $4.10^{-4}$ mole | - | - | - |
| 3,6-diméthyl-1H-pyrazolo[5,1-c][1,2,4]triazole | - | - | - | - | $4.10^{-4}$ mole | - | - |
| 2-(2,4-diamino-phénoxy)-éthanol ; chlorhydrate | - | - | - | - | - | $4.10^{-4}$ mole | - |
| 3-amino 2-chloro 6-méthyl phénol ; chlorhydrate | - | - | - | - | - | - | $4.10^{-4}$ mole |
| Support de teinture | (2) | (2) | (2) | (2) | (2) | (2) | (2) |
| Eau déminéralisée q.s.p. | 100 g | 100 g | 100 g | 100 g | 100 g | 100 g | 100 g |

(2) : Support de teinture pH 9,5

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en $C_8$-$C_{10}$ polyglucoside en solution aqueuse à 60% | 3,6 g M.A |
| Alcool benzylique | 2,0 g |
| Polyéthylène glycol à 8 motifs d'oxyde d'éthylène | 3,0 g |
| $NH_4Cl$ | 4,32 g |
| Ammoniaque à 20% de $NH_3$ | 2,94 g |

Au moment de l'emploi, chaque composition est mélangée avec un poids égal d'eau oxygénée à 20 volumes (6 % en poids). On obtient un pH final de 9,5.

Chaque mélange obtenu est appliqué sur des mèches de cheveux gris à 90 % de blancs. Après 30 minutes de pose, les mèches sont rinçées, lavées avec un shampooing standard, rinçées à nouveau puis séchées.

| Exemple | 50 | 51 | 52 | 53 | 54 | 55 | 56 |
|---|---|---|---|---|---|---|---|
| **Nuance observée** | Rouge orangé | Rouge intense | Rouge intense | Gris rouge intense | Violet rouge intense | Bleu | Violet bleu intense |

**Exemples 57 à 70 : compositions tinctoriales à partir du chlorure de 3-{3-[4, 7-bis-(4-amino-phényl-[1,4,7]triazonan-1-yl]-propyl}-1-methyl-3H-imidazol-1-ium (10)**

Exemples 57 à 63 : teinture en milieu acide

Les compositions tinctoriales suivantes sont préparées :

| Exemple | 57 | 58 | 59 | 60 | 61 | 62 | 63 |
|---|---|---|---|---|---|---|---|
| Chlorure de 3-{3-[4,7-bis-(4-amino-phényl)-[1,4,7] triazonan-1-yl]-propyl}-1-méthyl-3H-imidazol-1-ium | $4.10^{-4}$ mole | $4.10^{-4}$ mole | $4.10^{-4}$ mole | $4.10^{-4}$ mole | $4.10^{-4}$ mole | $4.10^{-4}$ mole | $4.10^{-4}$ mole |
| Benzène-1,3-diol | $4.10^{-4}$ mole | - | - | - | - | - | - |
| 5-amino 2-méthyl phénol | - | $4.10^{-4}$ mole | - | - | - | - | - |
| 1H-indol-6-ol | - | - | $4.10^{-4}$ mole | - | - | - | - |
| 2-amino pyridin-3-ol | - | - | - | $4.10^{-4}$ mole | - | - | - |
| 3,6-diméthyl-1H-pyrazolo[5,1-c] [1,2,4]triazole | - | - | - | - | $4.10^{-4}$ mole | - | - |
| 2-(2,4-diamino-phénoxy)-éthanol ; chlorhydrate | - | - | - | - | - | $4.10^{-4}$ mole | - |
| 3-amino 2-chloro 6-méthyl phénol ; chlorhydrate | - | - | - | - | - | - | $4.10^{-4}$ mole |
| Support de teinture | (1) | (1) | (1) | (1) | (1) | (1) | (1) |
| Eau déminéralisée q. s.p. | 100 g | 100 g | 100 g | 100 g | 100 g | 100 g | 100 g |

(1 ) : Support de teinture pH 7

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en $C_8$-$C_{10}$ polyglucoside en solution aqueuse à 60% | 3,6 g M.A |
| Alcool benzylique | 2,0 g |
| Polyéthylène glycol à 8 motifs d'oxyde d'éthylène | 3,0 g |
| $Na_2HPO_4$ | 0,28 g |
| $KH_2PO_4$ | 0,46 g |

Au moment de l'emploi, chaque composition est mélangée avec un poids égal d'eau oxygénée à 20 volumes (6 % en poids). On obtient un pH final de 7.

Chaque mélange obtenu est appliqué sur des mèches de cheveux gris à 90 % de blancs. Après 30 minutes de pose, les mèches sont rinçées, lavées avec un shampooing standard, rinçées à nouveau puis séchées.

| Exemple | 57 | 58 | 59 | 60 | 61 | 62 | 63 |
|---|---|---|---|---|---|---|---|
| **Nuance observée** | Brun orangé intense | Gris violet rouge intense | Brun rouge intense | Brun rouge intense | Orange | Gris intense | Gris violet intense |

Exemples 64 à 70 : teintures en milieu basique

Les compositions tinctoriales suivantes sont préparées :

| **Exemple** | **64** | **65** | **66** | **67** | **68** | **69** | **70** |
|---|---|---|---|---|---|---|---|
| Chlorure de 3-{3-[4,7-bis-(4-amino-phényl)-[1,4,7] triazonan-1-yl]-propyl}-1-méthyl-3H-imidazol-1-ium | $4.10^{-4}$ mole | $4.10^{-4}$ mole | $4.10^{-4}$ mole | $4.10^{-4}$ mole | $4.10^{-4}$ mole | $4.10^{-4}$ mole | $4.10^{-4}$ mole |
| Benzène-1,3-diol | $4.10^{-4}$ mole | - | - | - | - | - | - |
| 5-amino 2-méthyl phénol | - | $4.10^{-4}$ mole | - | - | - | - | - |
| 1H-indol-6-ol | - | - | $4.10^{-4}$ mole | - | - | - | - |
| 2-amino pyridin-3-ol | - | - | - | $4.10^{-4}$ mole | - | - | - |
| 3,6-diméthyl-1H-pyrazolo[5,1-c] [1,2,4]triazole | - | - | - | - | $4.10^{-4}$ mole | - | - |
| 2-(2,4-diamino-phénoxy)-éthanol ; chlorhydrate | - | - | - | - | - | $4.10^{-4}$ mole | - |
| 3-amino 2-chloro 6-méthyl phénol ; chlorhydrate | - | - | - | - | - | - | $4.10^{-4}$ mole |
| Support de teinture | (2) | (2) | (2) | (2) | (2) | (2) | (2) |
| Eau déminéralisée q.s.p. | 100 g | 100 g | 100 g | 100 g | 100 g | 100 g | 100 g |

(2) : Support de teinture pH 9,5

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en $C_8$-$C_{10}$ polyglucoside en solution aqueuse à 60% | 3,6 g M.A |
| Alcool benzylique | 2,0 g |
| Polyéthylène glycol à 8 motifs d'oxyde d'éthylène | 3,0 g |
| $NH_4Cl$ | 4,32 g |
| Ammoniaque à 20% de $NH_3$ | 2,94 g |

Au moment de l'emploi, chaque composition est mélangée avec un poids égal d'eau oxygénée à 20 volumes (6 % en poids). On obtient un pH final de 9,5.

Chaque mélange obtenu est appliqué sur des mèches de cheveux gris à 90 % de blancs. Après 30 minutes de pose, les mèches sont rinçées, lavées avec un shampooing standard, rinçées à nouveau puis séchées.

| Exemple | 64 | 65 | 66 | 67 | 68 | 69 | 70 |
|---|---|---|---|---|---|---|---|
| Nuance observée | Brun intense | Violet intense | Rouge intense | Gris rouge intense | Violet rouge intense | Bleu intense | Violet intense |

## Revendications

**1.** Dérivés de triazacyclononane substitué sur l'un au moins des atomes d'azote par un groupement 4'-aminophényle substitué ou non substitué.

**2.** Dérivés selon la revendication 1 de formule (I) suivante et leurs sels d'addition :

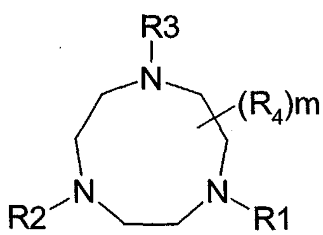

(I)

dans laquelle :

- $R_1$, $R_2$ et $R_3$ représentent :
  - un atome d'hydrogène ;
  - un radical alkyle pouvant être saturé ou insaturé, non substitué ou substitué par un ou plusieurs radicaux carboxyle, alkylcarbonyle, alcoxycarbonyle, carbamoyle, mono- ou di-alkylcarbamoyle, alkylsulfonyle, trialkylammonium, N-alkylimidazolium, hétérocycliques à 4, 5, 6 ou 7 atomes, saturés ou insaturés, dont le ou les hétéroatomes sont choisis parmi l'azote, l'oxygène et le soufre ;
  - un radical $-CH_2-R'$ où R' est un radical alkyle pouvant être saturé ou insaturé, substitué par un ou plusieurs radicaux hydroxy, alcoxy, amino, mono- ou di-alkylamino, trialkylammonium, alkylcarbonylamino, alkylcarbonyloxy ;
  - un radical alkylcarbonyle pouvant être saturé ou insaturé, non substitué ou substitué par un ou plusieurs radicaux hydroxyle, alcoxy, amino, mono- ou di-alkylamino, alkylsulfonyle, carboxyle, alkylcarbonyle, alcoxycarbonyle, carbamoyle, mono- ou di-alkylcarbamoyle, trialkylammonium, N-alkylimidazolium, hétérocycliques à 4, 5, 6 ou 7 atomes, saturés ou insaturés, dont le ou les hétéroatomes sont choisis parmi l'azote, l'oxygène et le soufre ;
  - un radical alcoxycarbonyle ;
  - un radical mono- ou di-alkylcarbamoyle ;
  - un radical carbamoyle ;
  - un radical carboxyle ;
  - un radical alkylsulfonyle ;
  - un radical arylsulfonyle ;
  - un radical correspondant à la formule (II) :

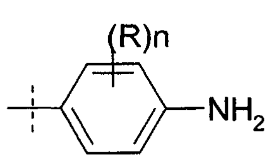

(II)

dans laquelle :

- R représente :

  - un radical alkyle pouvant être saturé ou insaturé, non substitué ou substitué par un ou plusieurs radicaux hydroxyle, amino, guanidinyle, trialkylammonium, N-alkylimidazolium, carboxyle, carbamoyle ;
  - un radical alcoxy pouvant être saturé ou insaturé, non substitué ou substitué par un ou plusieurs radicaux hydroxyle, amino, trialkylammonium, N-alkylimidazolium ;
  - un radical alcoxyalkyle ;
  - un radical hydroxyalcoxyalkyle ;
  - un radical aminoalkyle, l'amine pouvant être non substituée ou mono ou disubstituée par un radical alkyle, acétyle, hydroxyalkyle ;

- n est compris entre 0 et 4, étant entendu que lorsque n est supérieur à 1 alors les radicaux R peuvent être identiques ou différents ;

étant entendu qu'au moins un des radicaux $R_1$, $R_2$ ou $R_3$ est choisi parmi les radicaux de formule (II) ;

- $R_4$ représente :

  - un radical alkyle pouvant être saturé ou insaturé ;
  - un radical hydroxyalkyle ;
  - un radical alcoxyalkyle ;
  - un radical alkylcarbonyle ;
  - un radical hydroxyalcoxyalkyle ;
  - un radical aminoalkyle, l'amine pouvant être non substituée ou mono ou disubstituée par un radical alkyle, acétyle, hydroxyalkyle ;
  - un radical hydroxy et aminoalkyle ;
  - un radical carboxyle ;
  - un radical carboxyalkyle ;
  - un radical carbamoyle ;
  - un radical carbamoylalkyle ;
  - un radical alcoxycarbonyle ;
  - un radical mono- ou di-alkylaminocarbonyle ;

- m est compris entre 0 et 6, étant entendu que lorsque m est supérieur à 1 alors les radicaux $R_4$ peuvent être identiques ou différents.

**3.** Dérivés selon la revendication 2 de formule (I) dans laquelle deux des radicaux $R_1$, $R_2$ ou $R_3$ sont des radicaux correspondant à la formule (II).

**4.** Dérivés selon la revendication 2 ou 3 de formule (I) dans laquelle n est égal à 0 ou R est choisi parmi un radical alkyle ; un radical hydroxyalkyle ; un radical aminoalkyle ; un radical alcoxy ; un radical hydroxyalcoxy.

**5.** Dérivés selon la revendication 4 de formule (1) dans laquelle n est égal à 0 ou R est choisi parmi un radical méthyle ; un radical hydroxyméthyle ; un radical 1,2-dihydroxyéthyle.

**6.** Dérivés selon la revendication 4 ou 5 de formule (I) dans laquelle n est égal à 0 ou 1.

**7.** Dérivés selon l'une quelconque des revendications 2 à 6 de formule (I) dans laquelle le ou les radicaux $R_1$, $R_2$ ou $R_3$ différents des radicaux correspondant à la formule (II) sont choisis parmi un atome d'hydrogène ; un radical alkyle ; un radical alkylcarbonyle ; un radical alkylsulfonyle ; un radical $-CH_2-R'$ où R' est un radical alkyle substitué par un ou plusieurs radicaux hydroxy, alcoxy, amino, mono- ou di-alkylamino, trialkylammonium ; un radical alkyle substitué par un ou plusieurs radicaux trialkylammonium, N-alkylimidazolium ; un radical alkylcarbonyle substitué par un ou plusieurs radicaux N-alkylimidazolium ; un radical mono- ou di-alkylcarbamoyle.

**8.** Dérivés selon la revendication 7 de formule (I) dans laquelle le ou les radicaux $R_1$, $R_2$ ou $R_3$ différents des radicaux correspondant à la formule (II) sont choisis parmi un atome d'hydrogène ; un radical 2-hydroxyéthyle ; un radical

2,3-dihydroxy-propyle ; un radical 3-hydroxypropyle; un radical 2-aminoéthyle ; un radical 2-méthoxyéthyle ; un radical méthyle ; un radical éthyle ; un radical acétyle ; un radical méthylsulfonyle ; un radical N,N-diméthylcarbamoyle ; un radical 2-(N,N-diméthylamino)éthyle ; un radical (1-méthyl-imidazol-1-ium-3-yl)méthylcarbonyle ; un radical 3-(triméthylammonium)-2-hydroxy-propyle, un radical (1-méthyl-imidazol-1-ium-3-yl)propyle ; un radical 2-(triméthylammonium)-éthyle.

**9.** Dérivés selon l'une quelconque des revendications 2 à 8 de formule (I) dans laquelle m est égal à 0 ou $R_4$ est choisi parmi un radical alkyle ; un radical hydroxyalkyle ; un radical carboxyle ; un radical carbamoyle.

**10.** Dérivés selon la revendication 9 de formule (I) dans laquelle m est égal à 0 ou 1.

**11.** Dérivés selon l'une quelconque des revendications 2 et 4 à 10 choisis parmi le 1,4-bis-(4-amino-phényl)-[1,4,7] triazonane ; le 1-méthyl-4,7-bis-(4-amino-phényl)-[1,4,7]triazonane ; le 2-[4,7-bis-(4-amino-phényl)-[1,4,7]triazonan-1-yl]-éthanol ; le 1-éthyl-4,7-bis-(4-amino-phényl)-[1,4,7]triazonane ; le 3-[4,7-bis-(4-amino-phényl)-[1,4,7] triazonan-1-yl]-propane-1,2-diol ; le 1-acétyl-4,7-bis-(4-amino-phényl)-[1,4,7]triazonane ; le 1-(N,N-diméthyl-carbamoyl)-4,7-bis-(4-amino-phényl)-[1,4,7]triazonane ; le 1-(β-amino-éthyl)-4,7-bis-(4-amino-phényl)-[1,4,7]triazonane ; le 1-[β-(N,N-diméthyl-amino)-éthyl]-4,7-bis-(4-amino-phényl)-[1,4,7]triazonane ; le chlorure de 3-{2-[4,7-bis-(4-amino-phényl)-[1,4,7]triazonan-1-yl]-2-oxo-éthyl}-1-méthyl-3H-imidazol-1-ium ; le 1-(γ-hydroxy-propyl)-4,7-bis-(4-amino-phényl)-[1,4,7]triazonane ; le 1-(β-méthoxy-éthyl)-4,7-bis-(4-amino-phényl)-[1,4,7]triazonane ; le 1,4-bis-(4-amino-3-méthyl-phényl)-[1,4,7]triazonane ; le 1-méthyl-4,7-bis-(4-amino-3-méthyl-phényl)-[1,4,7]triazonane ; le 2-[4,7-bis-(4-amino-3-méthyl-phényl)-[1,4,7]triazonan-1-yl]-éthanol ; le 1 -éthyl-4,7-bis-(4-amino-3-méthyl-phényl)-[1,4,7]triazonane ; le 3-[4,7-bis-(4-amino-3-méthyl-phényl)-[1,4,7]triazonan-1-yl]-propane-1,2-diol ; le 1-acétyl-4,7-bis-(4-amino-3-méthyl-phényl)-[1,4,7]triazonane ; le 1-(N,N-diméthyl-carbamoyl)-4,7-bis-(4-amino-3-méthyl-phényl)-[1,4,7]triazonane ; le 1-(β-amino-éthyl)-4,7-bis-(4-amino-3-méthyl-phényl)-[1,4,7]triazonane ; le 1-[β-(N,N-diméthyl-amino)-éthyl]-4,7-bis-(4-amino-3-méthyl-phényl)-[1,4,7]triazonane ; le chlorure de 3-{2-[4,7-bis-(4-amino-3-méthyl-phényl)-[1,4,7]triazonan-1-yl]-2-oxo-éthyl}-1-méthyl-3H-imidazol-1-ium ; le 3-[4,7-bis-(4-amino-3-méthyl-phényl)-[1,4,7]triazonan-1-yl]-propan-1-ol ; le 1-(β-méthoxy-éthyl)-4,7-bis-(4-amino-3-méthyl-phényl)-[1,4,7]triazonane ; le 1,4,7-tris-(4-amino-phényl)-[1,4,7]triazonane ; le 1,4,7-tris-(4-amino-3-méthyl-phényl)-[1,4,7]triazonane ; le 1-(4-amino-phényl)-[1,4,7]triazonane ; le 1 ,4-diméthyl-7-(4-amino-phényl) -[1,4,7]triazonane ; le 1,4-bis-méthanesulfonyl-7-(4-amino-phényl)-[1,4,7]triazonane ; le 1,4-diacétyl-7-(4-amino-phényl)-[1,4,7]triazonane ; le 1,4-bis-(β-hydroxy-éthyl)-7-(4-amino-phényl)-[1,4,7]triazonane ; le 1-(4-amino-phényl)-4,7-bis-(γ-triméthyl-ammonium-β-hydroxy-propyl)-[1,4,7]triazonane ; le 1-(4-amino-3-méthyl-phényl)-[1,4,7] triazonane ; le 1,4-diméthyl-7-(4-amino-3-méthyl-phényl)-[1,4,7]triazonane ; le 1,4-bis-méthanesulfonyl-7-(4-amino-3-méthyl-phényl)-[1,4,7]triazonane ; le 1,4-diacétyl-7-(4-amino-3-méthyl-phényl)-[1,4,7]triazonane ; le 1,4-bis-(β-hydroxy-éthyl)-7-(4-amino-3-méthyl-phényl)-[1,4,7]triazonane ; le chlorure de 1,4-bis-(γ-triméthyl-ammonium-β-hydroxy-propyl)-7-(4-amino-3-méthyl-phényl)-[1,4,7]triazonane ; le chlorure de 3-{3-[4,7-bis-(4-amino-phényl)-[1,4,7]triazonan-1-yl]-propyl}-1-méthyl-3H-imidazol-1-ium ; le chlorure de 3-{3-[4,7-bis-(4-amino-3-méthyl-phényl)-[1,4,7]triazonan-1-yl]-propyl}-1-méthyl-3H-imidazol-1-ium ; le chlorure de {2-[4,7-bis-(4-amino-phényl)-[1,4,7]triazonan-1-yl]-éthyl]-triméthyl-ammonium ; le chlorure de {2-[4,7-bis-(4-amino-3-méthyl-phényl) -[1,4,7]triazonan-1-yl]-éthyl}-triméthyl-ammonium.

**12.** Dérivés selon la revendication 11 de formule (I) choisis parmi le 1-(4-amino-phényl)-[1,4,7]triazonane ; le 1,4-bis-(4-amino-phényl)-[1,4,7]triazonane ; le 1,4,7-tris-(4-amino-phényl)-[1,4,7]triazonane ; le 2-[4,7-bis-(4-amino-phényl)-[1,4,7]triazonan-1-yl]-éthanol et le chlorure de 3-{3-[4,7-bis-(4-amino-phényl)-[1,4,7]triazonan-1-yl]-propyl}-1-méthyl-3H-imidazol-1-ium.

**13.** Utilisation à titre de base d'oxydation pour la teinture d'oxydation des fibres kératiniques d'un dérivé tel que défini dans l'une quelconque des revendications 1 à 12.

**14.** Composition pour la teinture des fibres kératiniques comprenant, dans un milieu approprié pour la teinture, au moins un dérivé tel que défini dans l'une quelconque des revendications 1 à 12 à titre de base d'oxydation.

**15.** Composition selon la revendication 14 dans laquelle la quantité en dérivés tels que définis dans l'une quelconque des revendications 1 à 12 est comprise entre 0,001 et 10 % en poids du poids total de la composition tinctoriale.

**16.** Composition selon la revendication 14 ou 15 comprenant de plus un coupleur choisi parmi les méta-phénylènediamines, les méta-aminophénols, les méta-diphénols, les coupleurs naphtaléniques, les coupleurs hétérocycliques et leurs sels d'addition.

**17.** Composition selon la revendication 16 dans laquelle la quantité de chacun des coupleurs est comprise entre 0,001 et 10 % en poids du poids total de la composition tinctoriale.

**18.** Composition selon l'une quelconque des revendications 14 à 17 comprenant une base additionnelle choisie parmi les para-phénylènediamines autres que les dérivés tels que définis dans l'une quelconque des revendications 1 à 12, les bis-phénylalkylènediamines, les para-aminophénols, les bis-para-aminophénols, les ortho-aminophénols, les ortho-phénylènediamines, les bases hétérocycliques et leurs sels d'addition.

**19.** Composition selon la revendication 18 dans laquelle la quantité de chacune des bases d'oxydation additionnelles est comprise entre 0,001 et 10 % en poids du poids total de la composition tinctoriale.

**20.** Procédé de teinture des fibres kératiniques, **caractérisé en ce qu'**une composition telle que définie dans l'une quelconque des revendications 14 à 19 est appliquée sur les fibres kératiniques en présence d'un agent oxydant pendant un temps suffisant pour développer la coloration désirée.

**21.** Procédé selon la revendication 20 dans lequel l'agent oxydant est choisi parmi le peroxyde d'hydrogène, le peroxyde d'urée, les bromates de métaux alcalins, les persels, les peracides et les enzymes oxydases.

**22.** Dispositif à plusieurs compartiments dans lequel un premier compartiment contient une composition tinctoriale telle que définie dans l'une quelconque des revendications 14 à 19 et un deuxième compartiment contient un agent oxydant.

**23.** Dérivés intermédiaires de triazacyclononane substitué sur l'un au moins des atomes d'azote par un groupement 4'-nitrophényle substitué ou non substitué.

**24.** Dérivés selon la revendication 23 de formule (IV) suivante et leurs sels d'addition :

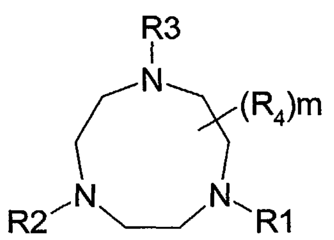

(IV)

dans laquelle :

- $R_1$, $R_2$ et $R_3$ représentent :
  - un atome hydrogène ;
  - un radical alkyle pouvant être saturé ou insaturé, non substitué ou substitué par un ou plusieurs radicaux carboxyle, alkylcarbonyle, alcoxycarbonyle, carbamoyle, mono- ou di-alkylcarbamoyle, alkylsulfonyle, trialkylammonium, N-alkylimidazolium, hétérocycliques à 4, 5, 6 ou 7 atomes, saturés ou insaturés, dont le ou les hétéroatomes sont choisis parmi l'azote, l'oxygène et le soufre ;
  - un radical $-CH_2-R'$ où R' est un radical alkyle pouvant être saturé ou insaturé, substitué par un ou plusieurs radicaux hydroxy, alcoxy, amino, mono- ou di-alkylamino, trialkylammonium, alkylcarbonylamino, alkylcarbonyloxy ;
  - un radical alkylcarbonyle pouvant être saturé ou insaturé, non substitué ou substitué par un ou plusieurs radicaux hydroxyle, alcoxy, amino, mono- ou di-alkylamino, alkylsulfonyle, carboxyle, alkylcarbonyle, alcoxycarbonyle, carbamoyle, mono- ou di-alkylcarbamoyle, trialkylammonium, N-alkylimidazolium, hétérocycliques à 4, 5, 6 ou 7 atomes, saturés ou insaturés, dont le ou les hétéroatomes sont choisis parmi l'azote, l'oxygène et le soufre ;
  - un radical alcoxycarbonyle ;
  - un radical mono- ou di-alkylcarbamoyle ;
  - un radical carbamoyle ;

- un radical carboxyle ;
- un radical alkylsulfonyle ;
- un radical arylsulfonyle ;
- un radical correspondant à la formule (V) :

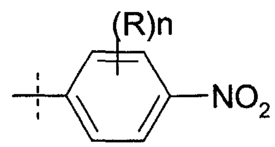

(V)

dans laquelle :

- R représente :

  - un radical alkyle pouvant être saturé ou insaturé, non substitué ou substitué par un ou plusieurs radicaux hydroxyle, amino, guanidinyle, trialkylammonium, N-alkylimidazolium, carboxyle, carbamoyle ;
  - un radical alcoxy pouvant être saturé ou insaturé, non substitué ou substitué par un ou plusieurs radicaux hydroxyle, amino, trialkylammonium, N-alkylimidazolium ;
  - un radical alcoxyalkyle ;
  - un radical hydroxyalcoxyalkyle ;
  - un radical aminoalkyle, l'amine pouvant être non substituée ou mono ou disubstituée par un radical alkyle, acétyle, hydroxyalkyle ;

- n est compris entre 0 et 4, étant entendu que lorsque n est supérieur à 1 alors les radicaux R peuvent être identiques ou différents ;

étant entendu qu'au moins un des radicaux $R_1$, $R_2$ ou $R_3$ est choisi parmi les radicaux de formule (II) ;

- $R_4$ représente :

  - un radical alkyle pouvant être saturé ou insaturé ;
  - un radical hydroxyalkyle ;
  - un radical alcoxyalkyle ;
  - un radical alkylcarbonyle ;
  - un radical hydroxyalcoxyalkyle ;
  - un radical aminoalkyle, l'amine pouvant être non substituée ou mono ou disubstituée par un radical alkyle, acétyle, hydroxyalkyle ;
  - un radical hydroxy et aminoalkyle ;
  - un radical carboxyle ;
  - un radical carboxyalkyle ;
  - un radical carbamoyle ;
  - un radical carbamoylalkyle ;
  - un radical alcoxycarbonyle ;
  - un radical mono- ou di-alkylaminocarbonyle ;

- m est compris entre 0 et 6, étant entendu que lorsque m est supérieur à 1 alors les radicaux $R_4$ peuvent être identiques ou différents.

Office européen des brevets

# RAPPORT DE RECHERCHE EUROPEENNE

Numéro de la demande
EP 04 29 2289

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| Y,D | JP 2002 255763 A (KAO CORP) 11 septembre 2002 (2002-09-11) * page 4 * | 1-24 | C07D521/00 |
| P,Y | -& WO 03/086336 A (KAO CORP) 23 octobre 2003 (2003-10-23) * abrégé * ----- | 1-24 | |
| D,Y | US 6 165 230 A (HENKEL KGAA) 26 décembre 2000 (2000-12-26) * revendications 1,7 * ----- | 1-24 | |
| Y | FR 2 828 488 A (OREAL) 14 février 2003 (2003-02-14) * revendication 1 * ----- | 1-24 | |
| Y | WO 01/68043 A (OREAL ; VIDAL LAURENT (FR); TERRANOVA ERIC (FR); SABELLE STEPHANE (FR)) 20 septembre 2001 (2001-09-20) * revendications 1,6 * ----- | 1-24 | |

DOMAINES TECHNIQUES RECHERCHES (Int.Cl.7)

C07D
A61K

Le présent rapport a été établi pour toutes les revendications

| Lieu de la recherche | Date d'achèvement de la recherche | Examinateur |
|---|---|---|
| La Haye | 24 janvier 2005 | Seitner, I |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

1

EPO FORM 1503 03.82 (P04C02)

**ANNEXE AU RAPPORT DE RECHERCHE EUROPEENNE RELATIF A LA DEMANDE DE BREVET EUROPEEN NO.** EP 04 29 2289

La présente annexe indique les membres de la famille de brevets relatifs aux documents brevets cités dans le rapport de recherche européenne visé ci-dessus.
*Lesdits members sont contenus au fichier informatique de l'Office européen des brevets à la date du*
Les renseignements fournis sont donnés à titre indicatif et n'engagent pas la responsabilité de l'Office européen des brevets.

24-01-2005

| Document brevet cité au rapport de recherche | Date de publication | Membre(s) de la famille de brevet(s) | Date de publication |
|---|---|---|---|
| JP 2002255763 A | 11-09-2002 | WO 03086336 A1 | 23-10-2003 |
| | | EP 1495751 A1 | 12-01-2005 |
| WO 03086336 A | 23-10-2003 | JP 2002255763 A | 11-09-2002 |
| | | WO 03086336 A1 | 23-10-2003 |
| | | EP 1495751 A1 | 12-01-2005 |
| US 6165230 A | 26-12-2000 | DE 19707545 A1 | 27-08-1998 |
| | | AT 237598 T | 15-05-2003 |
| | | AU 731108 B2 | 22-03-2001 |
| | | AU 6722998 A | 18-09-1998 |
| | | BR 9807857 A | 22-02-2000 |
| | | CA 2281500 A1 | 03-09-1998 |
| | | CN 1248250 T | 22-03-2000 |
| | | DE 59807968 D1 | 22-05-2003 |
| | | WO 9838175 A1 | 03-09-1998 |
| | | EP 0966449 A1 | 29-12-1999 |
| | | ES 2196554 T3 | 16-12-2003 |
| | | HU 0001491 A2 | 28-09-2000 |
| | | JP 2001513766 T | 04-09-2001 |
| | | NO 994115 A | 25-08-1999 |
| | | PL 335025 A1 | 27-03-2000 |
| | | SK 115599 A3 | 16-05-2000 |
| FR 2828488 A | 14-02-2003 | FR 2828488 A1 | 14-02-2003 |
| | | EP 1417184 A1 | 12-05-2004 |
| | | WO 03014093 A1 | 20-02-2003 |
| WO 0168043 A | 20-09-2001 | FR 2806299 A1 | 21-09-2001 |
| | | AU 774341 B2 | 24-06-2004 |
| | | AU 3938801 A | 24-09-2001 |
| | | BR 0105802 A | 26-03-2002 |
| | | CA 2373670 A1 | 20-09-2001 |
| | | CN 1372459 T | 02-10-2002 |
| | | EP 1200052 A2 | 02-05-2002 |
| | | WO 0168043 A2 | 20-09-2001 |
| | | HU 0201544 A2 | 28-08-2002 |
| | | JP 2003526647 T | 09-09-2003 |
| | | PL 352011 A1 | 14-07-2003 |
| | | RU 2223743 C2 | 20-02-2004 |
| | | US 2003093866 A1 | 22-05-2003 |

EPO FORM P0460

Pour tout renseignement concernant cette annexe : voir Journal Officiel de l'Office européen des brevets, No.12/82